(19) Europäisches Patentamt
European Patent Office
Office européen des brevets

(11) EP 1 015 457 B1

(12) **EUROPEAN PATENT SPECIFICATION**

(45) Date of publication and mention of the grant of the patent:
**26.11.2003 Bulletin 2003/48**

(21) Application number: **98939782.3**

(22) Date of filing: **07.08.1998**

(51) Int Cl.$^7$: **C07D 491/04**, A61K 31/55

(86) International application number:
**PCT/HU98/00076**

(87) International publication number:
**WO 99/007708 (18.02.1999 Gazette 1999/07)**

(54) **1,3-DIOXOLO/4,5-H//2,3/BENZODIAZEPINE DERIVATIVES AS AMPA/KAINATE RECEPTOR INHIBITORS**

1-3 DIOXOLO/4.5-H//2,3-BENZODIAZEPIN DERIVATE ALS AMPA/KAINATE-REZEPTOR-HEMMER

DERIVES DE 1,3-DIOXOLO/4,5-H//2,3/BENZODIAZEPINE UTILISES COMME INHIBITEURS DES RECEPTEURS AMPA/KAINATE

(84) Designated Contracting States:
**AT BE CH DE DK ES FI GB GR LI NL PT SE**
Designated Extension States:
**LT LV RO SI**

(30) Priority: **12.08.1997 HU 9701382**
**12.08.1997 HU 9701383**

(43) Date of publication of application:
**05.07.2000 Bulletin 2000/27**

(73) Proprietor: **EGIS GYOGYSZERGYAR RT.**
**1106 Budapest (HU)**

(72) Inventors:
- **BARKOCZY, Júzsef**
  **H-1016 Budapest (HU)**
- **CSELENYAK, Judit**
  **H-1124 Budapest (HU)**
- **RATKAI, Zoltán**
  **H-1101 Budapest (HU)**
- **SIMIG, Gyula**
  **H-1126 Budapest (HU)**
- **BALAZS, Lászlo**
  **H-1088 Budapest (HU)**
- **DOMAN, Imre**
  **H-1135 Budapest (HU)**
- **KOTAY NAGY, Péter**
  **H-2600 Vác (HU)**
- **GREFF, Zoltán**
  **H-1028 Budapest (HU)**
- **SERES, Péter**
  **H-1153 Budapest (HU)**
- **SZABO, Géza**
  **H-1054 Budapest (HU)**
- **GACSALYI, István**
  **H-1021 Budapest (HU)**
- **GIGLER, Gábor**
  **H-1067 Budapest (HU)**
- **GYERTYAN, István**
  **H-1165 Budapest (HU)**
- **LEVAY, György**
  **H-2092 Budakeszi (HU)**
- **KOVACS, Attila**
  **H-2510 Dorog (HU)**
- **SIMO, Annamária**
  **H-1137 Budapest (HU)**
- **SZABADOS, Tamás**
  **H-1108 Budapest (HU)**
- **EGYED, András**
  **H-1145 Budapest (HU)**
- **VEGH, Miklos**
  **H-1068 Budapest (HU)**
- **TIHANYI, Károly**
  **1171 Budapest (HU)**

(74) Representative: **Beszédes, Stephan G., Dr.**
**Patentanwalt,**
**Münchener Strasse 80a**
**85221 Dachau (DE)**

(56) References cited:

| | |
|---|---|
| **EP-A- 0 492 485** | **EP-A- 0 699 677** |
| **EP-A- 0 699 678** | **WO-A-92/11262** |
| **WO-A-95/01357** | **WO-A-96/04283** |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- **SOLYOM: "", PHARMAZIE, , 2001, vol. 56, no. , pages 62 to 66**

Remarks:

The file contains technical information submitted after the application was filed and not included in this specification

## Description

The invention refers to novel 1,3-dioxolo/4,5-h//2,3/benzodiazepine derivatives, a pharmaceutical composition containing the same, and a process for the preparation of the active ingredient as well as the use of the above compounds.

More specifically, the invention refers to novel 1,3-dioxolo/4,5-h//2,3/benzodiazepine derivatives of the formula I

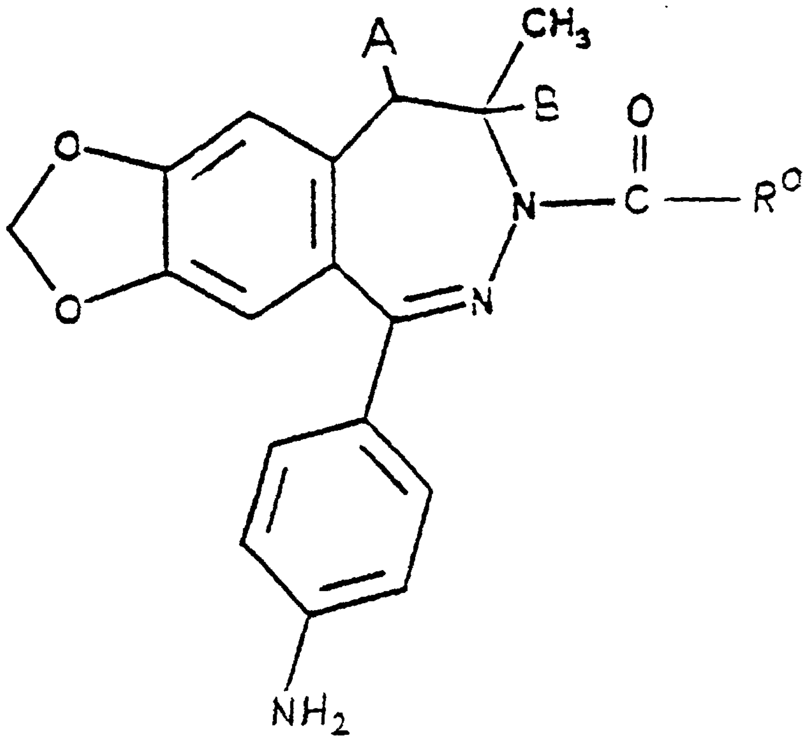

I,

a) A and B represent hydrogen or together form a valence bond
and

$R^0$ stands for a group of formula $-(CH_2)_m-R$,
wherein

R represents a halogen atom or a group of formula $-NR^3R^4$,
wherein

$R^3$ represents hydrogen and
$R^4$ means $C_{3-6}$ cycloalkyl, $C_{1-4}$ alkoxy, amino or $C_{1-4}$ alkyl, which latter is substituted by a saturated heterocyclic group having 5 or 6 members and comprising 1 nitrogen atom or 1 nitrogen atom and 1 oxygen atom as the heteroatom(s),
$R^3$ and $R^4$ mean, independently from each other, $C_{3-6}$ cycloalkyl, $C_{1-4}$ alkoxy, amino or $C_{1-4}$ alkyl, which latter is substituted by a saturated heterocyclic group having 5 or 6 members and comprising 1 nitrogen atom or 1 nitrogen atom and 1 oxygen atom as the heteroatom(s),
or
$R^3$ and $R^4$ together form with the adjacent nitrogen atom and optionally with an oxygen atom a saturated heterocyclic group having 5 or 6 members
and

m is 0, 1 or 2
or

b) A and B together form a valence bond,

$R^0$ stands for a group of formula $-(CH_2)_m-R$, wherein

R additionally to the meanings defined under a) alternatively represents a group of formula $-NR^3R^4$, wherein

$R^3$ and $R^4$ mean, independently from each other, hydrogen or unsubstituted $C_{1-4}$ alkyl
and

m is 0
or

c) A and B represent hydrogen,

$R^0$ stands for a group of formula $-(CH_2)_m-R$, wherein

R additionally to the meanings defined under a) alternatively represents a group of formula $-NR^3R^4$, wherein

$R^3$ means a hydrogen atom and
$R^4$ stands for an unsubstituted $C_{2-4}$ alkyl group
and

m is 0,

and pharmaceutically suitable acid addition salts thereof.

Several 2,3-benzodiazepine derivatives having biological activity are known.

Tofisopam i.e. 1-(3,4-dimethoxyphenyl)--5-ethyl-7,8-dimethoxy-4-methyl-5H-2,3--benzodiazepine having anxiclytic effect is known from HU-P Nc. 155 572 and GB-P No. 1 202 579, respectively. The known compound does not comprise the ring system 1,3-dioxolo/4,5-h//2,3/benzodiazepine.

From HU-P No. 186 760, 7,8-dihydro-8--methyl-9H-1,3-dioxolo/4,5-h//2,3/benzodiazepine derivatives having effect on the central nervous system are known, among others. The known compounds are prepared by reducing the corresponding 8-methyl-9H-1,3-dioxolo/4,5-h//2,3/benzodiazepine derivative.

Various substituted 8-methyl-9H-1,3--dioxolo/4,5-h//2,3/benzodiazepine derivatives are known from HU-P No. 191 698 and the corresponding GB-P No. 2 162 184. The known compounds have antiaggressive and anxiolytic activities.

A novel process for the preparation of partly new 8-methyl-9H-1,3-dioxolo/4,5-h//2,3/benzodiazepine derivatives having antiaggressive activity is known from HU-P No. 191 702. According to the novel process, the suitably substituted 2-acetonyl-4,5--methylenedioxybenzophenone is reacted with an excess of hydratine hydrate.

Further 7,8-dihydro-8-methyl-9H-1,3--dioxolo/4,5-h//2,3/benzodiazepine derivatives having antidepressant and antiparkinsonian activities are known from HU-P No. 206 719.

Some of the 2,3-benzodiazepine derivatives elicit their effect through the non-competitive inhibition of the AMPA/kainate receptors /Donevan, S.D. et al., J. Pharmacol. Exp. Ther., 271, 25-29 (1994)/.

From the literature it is known that AMPA/kainate receptors play an important role in the acute and chronic diseases of the central nervous system. Through the inhibition of these receptors, muscle relaxant, neuro-protective and anticonvulsive effects can be achieved /Vizi, E.S. et al., CNS Drug Reviews, 2, 91-126 (1996); Lees, G.L., CNS Drugs, 5, 51-74 (1996)/.

From WO 96/04283 there have been known 3-substituted 3H-2,3-benzodiazepine derivatives of general formula

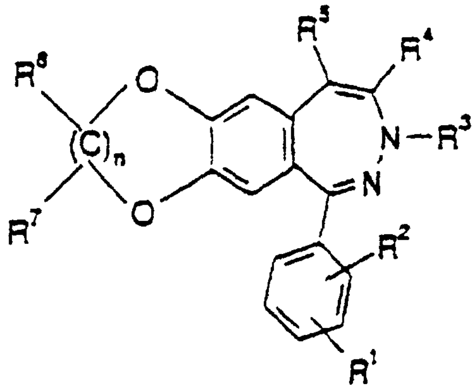

(A)

in which $R^1$ und $R^2$ are identical or different and mean hydrogen, $C_1$-$C_6$-alkyl, nitro, halogen, the group $-NR^8R^9$ or O-$C_{1-4}$-alkyl or $-CF_3$, $R^3$ stands for the group

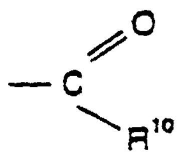

,

$R^4$ represents $C_1$-$C_6$-alkyl, optionally substituted, $R^5$ means hydrogen or $C_1$-$C_6$-alkyl, optionally substituted, $R^6$ and $R^7$ are identical or different and stand for hydrogen, $C_1$-$C_6$-alkyl, optionally substituted, or aryl, optionally substituted , $R^8$ and $R^9$ are identical or different and represent hydrogen, $C_1$-$C_6$-alkyl or the group

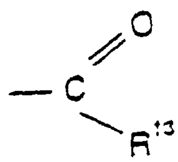

,

$R^{10}$ means hydrogen, $C_1$-$C_6$-alkyl, optionally substituted, aryl, optionally substituted, the group $-NR^{11}R^{12}$, -O-$C_{1-6}$-alkyl, $C_{3-7}$-cycloalkyl, $C_{2-6}$-alkenyl or -O-$C_{3-7}$-cycloalkyl, $R^{11}$ and $R^{12}$ are identical or different and stand for hydrogen, $C_1$-$C_6$-alkyl, optionally substituted, or aryl, optionally substituted; $R^{13}$ means $C_1$-$C_6$-alkyl and n stands for 1, 2 or 3, and their physiologically tolerated salts and isomers. Specifically also compounds in which $R^1$ is 4-$NH_2$, $R^2$ is hydrogen, $R^3$ is alkoxycarbonyl ($R^{10}$ being alkoxy), such as ethoxycarbonyl or methoxycarbonyl, $R^4$ is methyl, $R^5$, $R^6$ and $R^7$ are hydrogen atoms and n is 1 have been disclosed (Examples 46 and 47). Furthermore the activity of these compounds regarding diseases of the central nervous system, such as the inhibition of the AMPA-receptors, has been described.

In WO 95/01357 there have been described enantiomers of 1- (4-nitrophenyl)-4-methyl-7,8--methylenedioxy-3,4-dihydro-5H-2,3-benzodiazepine of formula

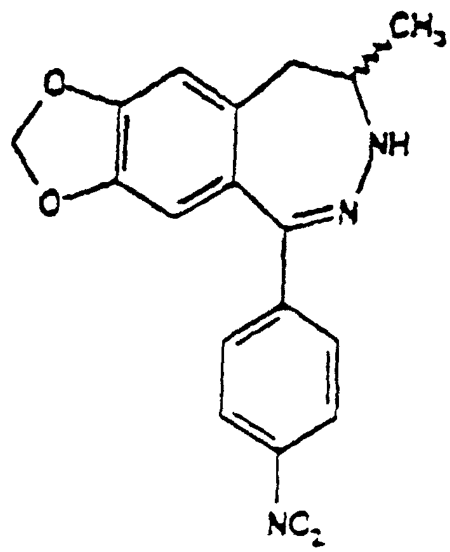

(B) .

However, these compounds compulsorily are not substituted on the ring nitrogen atom in the 3 position but contain a hydrogen atom. Moreover the activity of these compounds regarding diseases of the central nervous system, such as the inhibition of the AMPA--receptors, has been described.

From EP 699 678 there has been known compounds of general formula

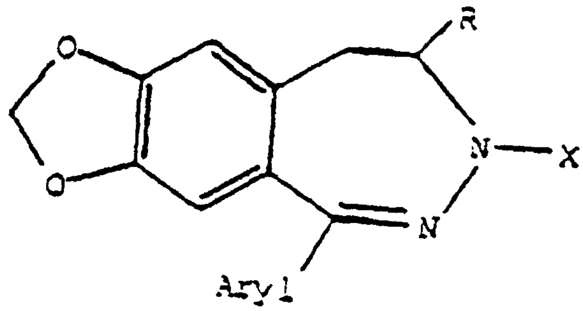

(C) ,

in which R is methyl, X is acetyl and Aryl is p-aminophenyl or p-nitrophenyl, that with Aryl = p--aminophenyl in a physical form having an X-ray powder diffraction pattern with d spacings al 12.78, 9.48, 8.99, 8.64, 8.23, 6.39, 6.27, 5.73, 4.01 and 3.96 Å. However, these compounds compulsorily contain an unsubstituted acetyl group on the ring nitrogen atom in the 3 position. Furthermore for the compound in which Aryl stands for p-aminophenyl the activity regarding diseases of the central nervous system, such as the inhibition of the AMPA-receptors, has been described.

Moreover in EP 492 485 there have been described N-acyl-2,3-benzodiazepine derivatives of general formula

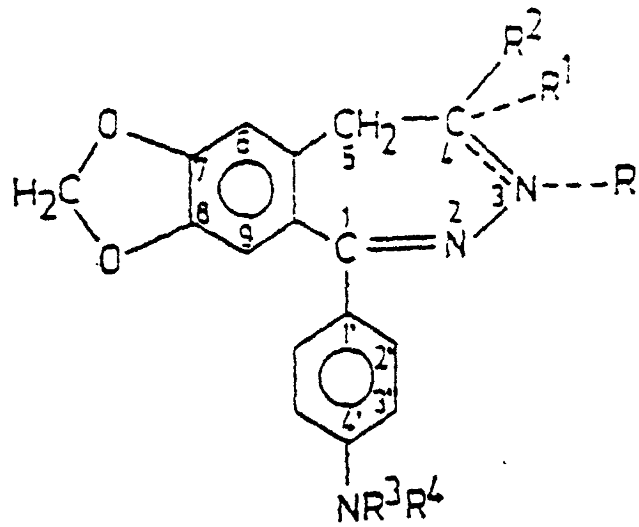

(D)

wherein R stands for a $C_{1-6}$ aliphatic acyl group, optionally substituted by a methoxy, cyano, carboxyl, amino, $C_{1-4}$ alkylamino, di($C_{1-4}$ alkyl)amino, pyrrolidino, phthalimido or phenyl group, or by one or more halogen(s); or R is a benzoyl, cyclopropanecarbonyl, $C_{1-5}$ alkylcarbamoyl or phenylcarbamoyl group; or R is absent when a double bond exists between the N(3) and C(4) atoms; $R^1$ means hydrogen; or $R^1$ is absent when a double bond exists between the N(3) and C(4) atoms; $R^2$ means a $C_{1-3}$ alkyl group; or $R^1$ and $R^2$ together stand for a methylene group and no double bond is present between the N(3) and C(4) atoms; $R^3$ means hydrogen or a $C_{1-4}$ aliphatic acyl group; $R^4$ represents hydrogen; a $C_{1-6}$ aliphatic acyl group optionally substituted by a methoxy, cyano, carboxyl, amino, $C_{1-4}$ alkylamino, di($C_{1-4}$ alkyl) amino, pyrrolidino, phthalimido or phenyl group or by one or more halogen(s); as well as a benzoyl, palmitoyl, cyclopropanecarbonyl, $C_{1-5}$ alkylcarbamoyl or phenylcarbamoyl group; and the dotted lines represent valence bonds, optionally being present, with the proviso that no double bond exists between the N(3) and C(4) atoms when both $R^3$ and $R^4$ stand for hydrogen, and their stereoisomers as well as acid-addition salts (where possible) of these compounds. Thus also compounds are comprised in which R is an acyl group, substituted by an amino or di-(alkyl)-amino group, $R^1$ is a hydrogen, $R^2$ is a $C_{1-3}$ alkyl group and $R^3$ and $R^4$ are hydrogen atoms. Furthermore the activity of these compounds on the central nervous system, particularly muscle-relaxant, anticonvulsive and neuro-protective action, has been described no mention being made of an inhibition of AMPA-receptors.

Furthermore from EP 699 677 there have been known dihydro-2,3-benzodiazepine derivatives of general formula

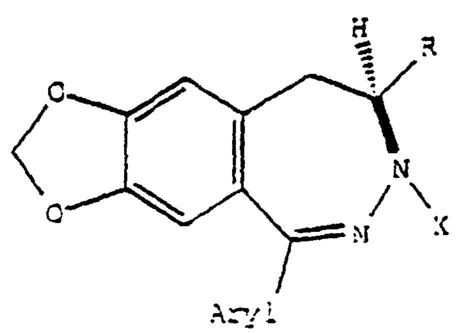

(E) ,

wherein R is hydrogen or a $C_1$-$C_{10}$ alkyl: and X is hydrogen, $C_1$-$C_{10}$ alkyl, acyl, aryl, carboxyl or a substituted derivative thereof, or a protecting group, or a pharmaceutically acceptable salt thereof. As acyl group for which X can stand specifically a carbamoyl or N-methylcarbamoyl group and as "aryl" specifically a p-nitrophenyl, p-aminophenyl or p-($C_1$-$C_6$ alkanoylamino)-phenyl have been mentioned. Furthermore also the activity of inhibiting the AMPA-receptors has been described without test data.

The aim of the invention is to prepare novel 2,3-benzodiazepine derivatives that are more effective and less toxic, respectively, than the known 2,3-benzodiazepine derivatives.

It was found that the above aim is achieved by the novel 1,3-dioxolo/4,5-h//2,3/benzodiazepine derivatives which have - due to their non-competitive AMPA/kainate effect - considerable muscle relaxant, neuroprotective and anticonvulsive activities. Thus, the novel compounds can be employed for the treatment of any diseases (such as epilepsy, diseases resulting in muscle spasm, various neurodegenerative diseases. stroke,) in which the inhibition of the AMPA/kainate receptors is favourable.

In the description and claims, in the definition of the substituents, under a halo atom primarily a fluoro, chloro, bromo or iodo atom, preferably a fluoro or a chloro atom is meant.

A $C_{1-4}$ alkyl group is a methyl, ethyl, n-propyl, isopropyl, n-butyl, sec.-butyl, tert.-butyl or isobutyl group. Preferably, a $C_{1-4}$ alkyl group is a methyl, an ethyl or an isopropyl group.

A $C_{1-4}$ alkoxy group is, primarily, a methoxy, ethoxy, n-propoxy, isopropoxy or n-butoxy group, preferably a methoxy group.

A $C_{3-6}$ cycloalkyl group is a cyclopropyl, cyclobutyl, cyclopentyl or cyclohexyl group, preferably a cyclopropyl group.

A saturated heterocyclic group having 5 or 6 members and comprising 1 nitrogen atom or a nitrogen atom and an oxygen atom as the heteroatom is preferably a morpholino group.

Under a pharmaceutically suitable acid addition salt an acid addition salt formed with a pharmaceutically suitable inorganic acid such as hydrochloric acid, hydrobromic acid, sulfuric acid, phosphoric acid, or with a pharmaceutically suitable organic acid such as formic acid, acetic acid, fumaric acid, maleic acid, lactic acid, malic acid, tartaric acid, succinic acid, citric acid, methanesulfonic acid, is meant.

The invention includes any isomers of the compounds of the formula I and the mixtures thereof.

Under the isomers of the compounds of the formula I - due to the presence of at least one chiral centre - both enantiomers, and - because of isomerisms that exist in case of certain substitutions - the isomers E and Z, diastereomers, tautomeric forms, and the mixtures thereof such as the racemate are meant.

A preferred subgroup of the compounds of the formula I consists of the 7,8-dihydro-8-methyl-9H-1,3--dioxolo/4,5-h//2,3/benzodiazepine derivatives,
wherein

A and B represent hydrogen atoms
and
the halogen atom which may be represented by R is a chloro atom
or
the cycloalkyl group for which

$R^3$ and/or $R^4$ may stand, is/are [a] cyclopropyl group(s) and

m is as above defined

and pharmaceutically suitable acid addition salts thereof.

Suitable 7,8-dihydro-8-methyl-9H-1,3-dioxolo/4,5-h//2,3/benzodiazepine derivatives are the following compounds of the formula I, wherein

A and B represent hydrogen atoms,
the alkyl group(s) for which $R^3$ and/or $R^4$ may stand is/are [a] $C_{1-2}$ alkyl group(s)
and
the heterocyclic group by which the alkyl group(s), for which $R^3$ and/or $R^4$ may stand, may be substituted or the heterocyclic group which may be formed by $R^3$ and/or $R^4$ with the adjacent nitrogen atom and with an oxygen atom is a morpholino group
and
m has a value of 0 or 1,

and pharmaceutically suitable acid addition salts thereof.

The especially preferred 7,8-dihydro--8-methyl-9H-1,3-dioxolo/4,5-h//2,3/benzodiazepine derivatives are the following compounds of the formula I, wherein

A and B represent hydrogen atoms,
$R^3$ represents a hydrogen atom,
$R^4$ stands for a cyclopropyl group or an amino group
and

m has a value of 0

and pharmaceutically suitable acid addition salts thereof.

Another preferred subgroup of the compounds of the invention consists of the 8-methyl-7H-1,3-clioxolo/4,5-h//2,3/benzodiazepine derivatives of the formula I, wherein

A and B together form a valence bond,
and
the halogen atom which may be represented by R is a chloro atom
or
the alkyl group(s) for which the heterocyclic group(s), by which the alkyl group(s), for which $R^3$ and/or $R^4$ may stand, may be substituted, is/are [a] morpholino group(s)
and
m has a value of 0, 1 or 2,

and pharmaceutically suitable acid addition salts thereof.

The 1,3-dioxolo/4,5-h//2,3/benzodiazepine derivatives of the formula I, wherein A, B and $R^0$ are as above defined are prepared as follows:

a) for the preparation of a compound of the formula I, wherein $R^0$ represents a group of the formula $-(CH_2)_m-R$, in which R stands for a halogen atom, m has a value of 0, 1 or 2 and A and B represent hydrogen atoms, the 7,8-dihydro-8-methyl-5-(4--nitrophenyl)-9H-1,3-dioxolo/4,5-h//2,3/benzodiazepine of the formula III

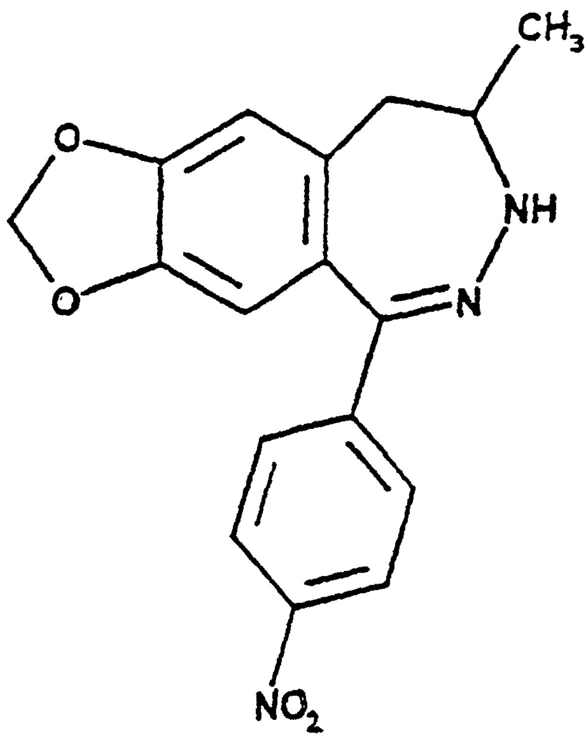

III

is reacted with a reagent of the formula VI

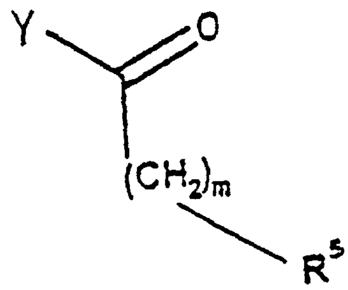

VI ,

wherein Y represents a leaving group and $R^5$ is a halogen atom and the obtained compound in which instead of the amino group on the phenyl ring of the formula I there is a nitro group and $R^0$, A and B are as defined in connection with the formula I, is transformed into a compound of the formula I by reduction;

or

b) for the preparation of a compound of the formula I, wherein $R^0$ represents a group of the formula $-(CH_2)_m-R$, wherein R stands for a group of the formula $-NR^3R^4$, in which $R^3$, $R^4$ and m are as defined in connection with formula I and A and B represent hydrogen atoms, the 7,8-dihydro-8--methyl-5-(4-nitrophenyl)-9H-1,3-dioxolo/4,5--h//2,3/benzodiazepine of the formula III is reacted with a reagent of the formula VI, wherein Y and $R^5$ represent, independently, a leaving group and m is as stated above, and the obtained benzodiazepine derivative of the formula IV

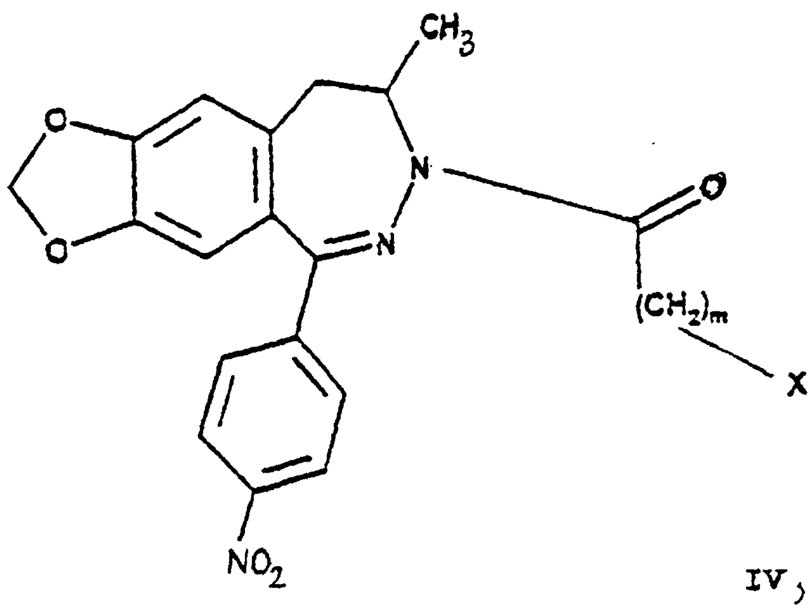

IV ,

wherein X stands for a leaving group, m is as stated above, is reacted with an amine of the formula VII

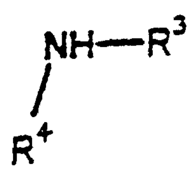

VII ,

wherein $R^3$ and $R^4$ are as stated above and the obtained compound in which instead of the amino group on the

phenyl ring of the formula I there is a nitro group and $R^0$, A and B are as defined in connection with the formula I, is transformed into a compound of the formula I by reduction;

or

c) for the preparation of a compound of the formula I, wherein $R^0$ stands for a group of the formula - $(CH_2)_m$-R, in which R represents a halogen atom, m has a value of 0, 1 or 2 and A forms together with B a valence bond, the 8-methyl-5-(4-nitrophenyl)-- 9H-1,3-dioxolo/9,5-h//2,3/benzodiazepine of the formula II

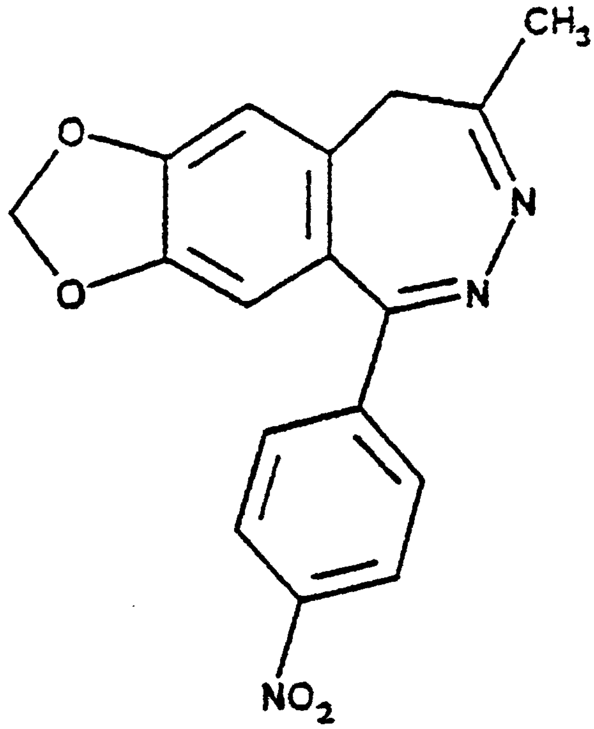

II

is reacted with an acylating agent of the formula IX

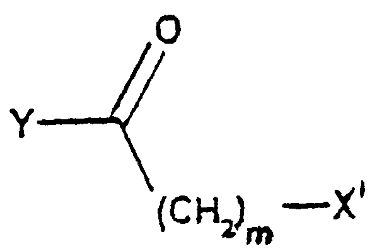

IX ,

wherein Y represents a leaving group, X' stands for a halogen atom and m has a value of 0, 1 or 2 and the obtained compound in which instead of the amino group on the phenyl ring of the formula I there is a nitro group and $R^0$, A and B are as defined in connection with the formula I, is transformed into a compound of the formula I by reduction;

or

d) for the preparation of a compound of the formula I, wherein $R^0$ stands for a group of the formula $-(CH_2)_m$-R, in which R represents a group of the formula $-NR^3R^4$, wherein $R^3$, $R^4$ and m are as defined in connection with the formula I and A forms together with B a valence bond, the 8-methyl-5-(4--nitrophenyl)-9H-1,3-dioxolo-/4,5--h// 2,3/benzodiazepine of the formula II is reacted with an acylating agent of the formula IX, wherein each of Y and X' represent, independently from each other, a leaving group and m is as stated above, and the obtained acylated compound of the formula VIII

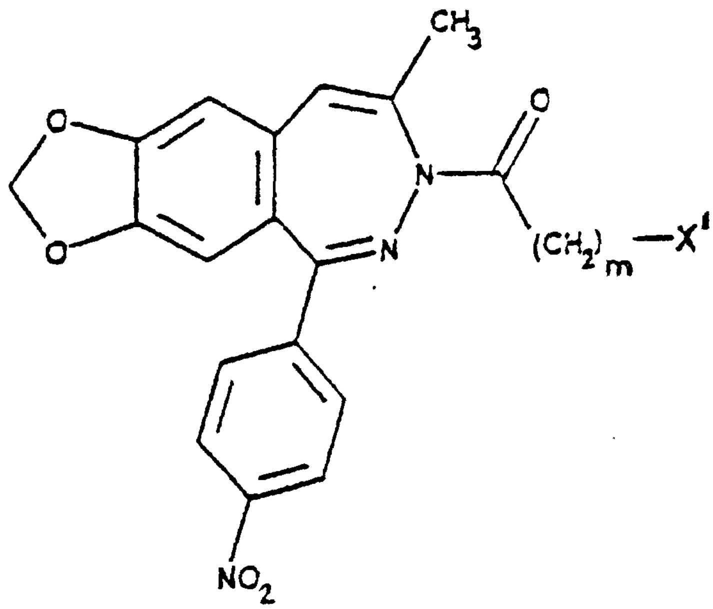

VIII,

wherein X' and m are as defined above, is reacted with an amine of the formula $HNR^3R^4$, wherein $R^3$ and $R^4$ are as stated above; and the obtained compound in which instead of the amino group on the phenyl ring of the formula I there is a nitro group and $R^0$, A and B are as defined in connection with the formula I, is transformed into a compound of the formula I by reduction;

and, if desired, an obtained base of the formula I is converted to a pharmaceutically suitable acid addition salt or liberated from the acid addition salt.

The reagent of the formula VI is an acylating agent, such as a carboxylic halide, a carboxylic anhydride, a carbonate ester, carbonyldiimidazole, an omega-halocarboxylic halide or an omega-halocarbonate ester. The acylation is carried out in the presence or absence of an acid binding agent and/or pyridine, at a temperature of -20 to +150 °C, in the presence or absence of an organic solvent.

The reaction of the benzodiazepine derivative of the formula IV and the amine of the formula VII is carried out in a manner known from the literature /Houben-Weyl: Methoden der Organischen Chemie, Band XI, Amine, G. Thieme Verlag, Stuttgart, 1957; S. Patai: The chemistry of amine group, Interscience Publishers, 1968/.

The acylation of the compound of the formula II with the acylating agent of the formula IX and the amination of the compound of the formula VIII with the amine of the formula $HNR^7R^8$ are performed in a similar manner as described above.

The nitro compounds of the formula I can be reduced in a manner known in itself to obtain the corresponding amino compound. The reduction can be carried out for example with tin(II) chloride or in the presence of a catalyse using a hydrogen source. For example, the catalyse can be Raney nickel, palladium or platinum oxide, the hydrogen source is, for example, hydrazine, hydrazine hydrate, formic acid, a trialkylammonium formate or an alkali metal formate.

If desired, a base of the formula I is reacted with an inorganic or organic acid to transform it into a pharmaceutically suitable acid addition salt, or the base of the formula I is libereted from the acid addition salt using a stronger base.

The starting compound 7,8-dihydro-8--methyl-5-(4-nitrophenyl)-9H-1,3-dioxolo/4,5-h//2,3/benzodiazepine of the formula III can be prepared by reducing 8-methyl--5-(4-nitrophenyl)-9H-1,3-dioxolo/4,5-h//2,3/benzodiazepine of the formula II in an analogous manner as described in the literature /Houben-Weyl: Methoden der Organischen Chemie, Band IV, Reduktion, G. Thieme Verlag, Stuttgart, 1989/ or using the processes known from HU-P No. 186 760.

The compound of the formula II can be prepared by the process known from HU-P No. 191 702.

The reagents of the formulae VI and IX as well as the amines of the formula VII are commercially available.

The pharmacological effect of the novel compounds of the formula I was studied by in vitro and in vivo methods. 8-Methyl-5--(4-aminophenyl)-9H-1,3-dioxolo/4,5-h//2,3/benzodiazepine (compound "A") known from HUP No. 191 698 and GB-P No. 2 162 184 was used as the reference substance.

In vitro determination of AMPA antagonist effect

PSI (inhibition of population spike) test

The field potentials (population spike) evoked by electric stimulation of the Shaffer collateral comissural pathway were measured in the CAl neurones of rat hippocampus. The population spike can be inhibited by AMPA/kainate antagonists. The non-cumulative $IC_{50}$ values are shown in Table I. /Tarnawa, I., Molnár, P., Gaál, L., Andrási, F.: Inhibition of hippocampal field potentials by GYKI 52466 in vitro and in vivo, Acta Physiol. Hung., 79(2), 163-9 (1992)/.

SD (spreading depression) test

The method is based on the phenomenon of spreading depression evoked by kainate in isolated retinal preparation of the chicken. The formation of spreading depression is inhibited (delayed) by AMPA/kainate antagonists. /Sheardown M.J.: The triggering of spreading depression in the chicken retina: a pharmacological study, Brain Res., 607(1-2), 189-194 (1993)/. The obtained $IC_{50}$ values are shown in Table I.

Table I

| Results obtained in tests suitable for the determination of in vitro AMPA antagonist effect | | |
|---|---|---|
| Compound (No. of Example) | Percent inhibition of population spike (10 micron) | SD[a] $IC_{50}$ in microM |
| 2 | 100 | 1.3 |
| 3 | 95 | 1.5 |
| 4 | 95 | no data |
| 6 | no data | 6.5 |
| 11 | no data | 2.8 |
| "A" | 58 | 9.5 |

[a] Spreading depression test.

As shown in Table I, the inhibitory effects of the novel compounds are significantly higher than that of reference compound "A".

In vivo assays

Muscle relaxant effect

The assay was done according to Hoppe in male NMRI mice weighing 20 to 25 g, with 10 animals in each group /Hoppe, J.O., J . Pharmacol. Exp. Ther., 100, 333 (1950)/. Following the ip. treatment of animals, the number of mice showing muscle weakness were recorded at every 10 minutes in the first hour and at half hour intervals afterwards. The animals falling off the 60° inclined screen within 30 seconds were considered positive. $ED_{50}$ values of the given compounds were determined at each time. The duration of effect was defined as the time of last reading when the effect was at least 30 %. The results obtained are summarized in Table II.

Table II

| Muscle relaxant effect | | |
|---|---|---|
| Compound (No. of Example) | Muscle relaxant $ED_{50}$[x] ip, in mg/kg | effect duration in hr |
| 2 | 21.1 | higher than 2 |
| 3 | 18.1 | 4 |
| "A" | 24.5 | 1 |

[x] determined at the time of maximal effect.

Although the muscle relaxant activity of the novel compounds are about the same as that of reference compound "A", the duration of action is significantly longer as shown in Table II.

Maximal electroshock test (MES)

Male NMRI mice weighing 20 to 30 g were used for the method of Swinyard et al. /Swinyard, E.A., Brown, W.C. and Goodman, L.S.: Comparative assays of antiepileptic drugs in mice and rats, J. Pharmacol., 106, 319 (1952) /. The animals, 10 in each group, were treated ip. either with various doses of the test substance or with vehicle. After 30 minutes, a 50 Hz, 40 mA electroshock was applied for 0.4 s through corneal electrodes. The number of animals that developed tonic extensor convulsion of the hind-limbs was registered, percent inhibition was calculated, and $ED_{50}$ values were determined by the method of Litchfield and Wilcoxon /Litchfield, J.T., Wilcoxon, F.A.: A simplified method of evaluating dose-effect experiments, J. Pharmacol. Exp. Ther., 96, 99 (1949)/ and summarized in Table III.

Audiogenic seizure (AS) test

The experiments were carried out by the slightly modified method of De Sarro et al. /De Sarro, G.B., Croucher, M.J. and Meldrum, B.S.: Anticonvulsant action of DS 103-282, Neuropharm., 23, 525 (1984)/. Groups of 8 male DBA/2j strain mice weighing 7 to 14 g were treated ip. with the test substance in 10 ml/kg volume. 15 minutes later, the animals were placed into a covered glass container (30 cm in diameter) and exposed to a 14 kHz 120 dB tone for 60 s at the most. Seizure response was assessed using the following scale: 0 = normal behaviour, 1 = wild running, 2 = clonus, 3 = tonic flexor seizure, 4 = tonic extensor seizure. The maximum response during the 60 s exposure was recorded for each animal. Lethality was also noted. The $ED_{50}$ values were determined by the method of Litchfield and Wilcoxon concerning the inhibition of clonic seizures and tonic extensor convulsions. The results are summarized in Table III.

Table III

Anticonvulsant effect following ip. treatment

| Compound (No. of Example) | $MES^{x}$ | $AS^{xx}$ | |
|---|---|---|---|
| | $ED_{50}$ in mg/kg | | |
| | | tonic | clonic |
| | | convulsion | |
| 2 | 4.6 | 1.6 | 2.5 |
| 3 | 3.7 | no data | no data |
| "A" | 6.9 | 3.6 | 4.3 |

x Inhibition of maximal electroshock.

xx Inhibition of sound induced seizure.

The novel compounds are significantly more effective at the inhibition of maximal electroshock and audiogenic seizure than the reference compound "A" as shown in Table III.

The compound of Example 6 has an approximate anticonvulsive $ED_{50}$ value of 10 mg/kg ip. in the MES test (not shown in Table III), while in 60 mg/kg dose it has no muscle relaxant effect in the inclined screen. In contrast, the anticonvulsive $ED_{50}$ value of the reference compound "A" is 6.9 mg/kg, however, at about 4.5 times higher dose, the reference compound produces about 50 % muscle relaxant effect, and at 60 mg/kg dose all the treated animals showed muscle relaxation. Since strong muscle relaxation may seriously limit the therapeutic application of a drug, the lack of muscle relaxant effect of some novel compounds of the invention provides potential advantage over reference compound "A" in the clinical use.

Global ischemia induced by magnesium chloride

The experiments were carried out as described by Berga et al. /Berga, P., Beckett, P.R., Roberts, D.J., Llenas, J., Massingham, R.: Synergistic interactions between piracetam and dihydroergocristine in some animal models of cerebral hypoxia and ischemia, Arzneim.--Forsch., 36, 1314-1320 (1986)/. Groups of 10 male NMRI mice weighing 20 to 25 g were treated ip. with the test substance in 10 mg/kg volume. After 30 minutes, saturated aqueous magnesium chloride solution was applied iv. (5 ml/kg) resulting in an immediate cardiac arrest. The elapsed time between the iv. injection and the last gasping was measured (gasping time). The means of the treated groups were expressed as percent of control. Statistical analysis was done by ANOVA followed by DUNCAN test. The dose resulting in 50 % descrease in gasping time ($ID_{50}$) was calculated by linear regression. The results are shown in Table IV.

Table IV

| Increase in gasping time in the magnesium chloride induced global ischemia test in mice | | | |
|---|---|---|---|
| Compound (No. of Example) | Dose in mg/kg ip. | Effect in % | $ID_{50}$ in mg/kg ip. |
| 2 | 30 | 61 | 13 |
| 3 | 30 | 52 | 27 |
| "A" | 30 | 55 | 30 |

From Table IV it can be seen that the novel compound of Example 16 is as effective at neuroprotection in 13 mg/kg dose as the reference compound "A" in 30 mg/kg dose.

Thus, the novel 8-substituted-9H--1,3-dioxolo/4,5-h//2,3/benzodiazepine derivatives of the formula I can be used as active ingredients of pharmaceutical compositions.

On the basis of the above test results, the novel compounds of the invention - due to their competitive AMPA/kainate antagonist property - have considerable muscle relaxant, neuroprotective and anticonvulsive effects. Consequently, the novel compounds can be used for the treatment of any disease such as epilepsy, diseases resulting in muscle spasm, neurodegenerative diseases, spates after stroke, migraine and vomiting, wherein the inhibition of the AMPA/kainate receptors may have a favourable effect.

Some compounds of the invention which possess considerable anticonvulsive and neuroprotective activities, while they have no or weak muscle relaxant effect, can be primarily applied as antiepileptics. In the course of their application, the lack of muscle relaxant action provides notable benefit over the known AMPA/kainate antagonist 2,3-benzodiazepine derivatives.

The pharmaceutical compositions of the invention contain a therapeutically active amount of a compound of the formula I or a pharmaceutically suitable acid addition salt thereof and one or more conventional carrier(s).

The pharmaceutical compositions of the invention are suitable for peroral, parenteral or rectal administration or for local treatment, and can be solid or liquid.

The solid pharmaceutical compositions suitable for peroral administration may be such as powders, capsules, tablets, film-coated tablets and microcapsules, and can comprise binding agents such as gelatine, sorbitol and poly (vinylpyrrolidone); filling agents such as lactose, glucose, starch and calcium phosphate; auxiliary substances for tabletting such as magnesium stearate, talc, poly (ethyleneglycol) and silica; wetting agents such as sodium laurylsulfate, as the carrier.

The liquid pharmaceutical compositions suitable for peroral administration may be solutions, suspensions or emulsions and can comprise e.g. suspending agents such as gelatine and carboxymethylcellulose; emulsifiers such as sorbitane monooleate; solvents such as water, oils, glycerol, propyleneglycol and ethanol; preservatives such as methyl p-hydroxybenzoate, as the carrier.

Pharmaceutical compositions suitable for parenteral administration consist of sterile solutions of the active ingredient, in general.

Dosage forms listed above as well as other dosage forms are known per se, see e.g. Remington's Pharmaceutical Sciences, 18th Edition, Mack Publishing Co., Easton, USA (1990).

The pharmaceutical compositions of the invention contain, in general, 0.1 to 95.0 per cent by mass of a compound of the formula I or a pharmaceutically suitable acid addition salt thereof. A typical dose for adult patients amounts to 0.1 to 20 mg of the compound of the formula I or a pharmaceutically suitable acid addition salt thereof, daily. The above dose can be administered in one or more portions. The actual dosage depends on many factors and is determined by the doctor.

The pharmaceutical compositions of the invention are prepared by admixing a compound of the formula I or a pharmaceutically suitable acid addition salt thereof to one or more carrier(s), and converting the mixture obtained to a pharmaceutical composition in a manner known per se. Useful methods are known from the literature, e.g. Remington's Pharmaceutical Sciences.

In the pharmaceutical compositions of the invention in the active ingredients the preferred meanings of the symbols in formula I are the same as above defined regarding the compounds of formula I as such.

Furthermore the invention refers to the use of the above 1,3-dioxolo[4,5-h][2,3]-benzodiazepines for preparing medicaments for the treatment of epilepsy or a neurodegenerative disease or a state after stroke.

The invention is further elucidated, in detail, by means of the following Examples.

A general process for reducing the nitro group of the starting compounds used in the following Examples 1 to 4 by catalytical hydrogenation

5.0 mmoles of the nitro compound are dissolved in a mixture of 100 $cm^3$ of dichloromethane and 100 $cm^3$ of methanol, and the solution is hydrogenized in the presence of 0.10 g of 10 % palladium/carbon catalyst at room temperature and $5.065 \times 10^5$ Pa pressure. Following the hydrogenization, the catalyst is filtered, the solvent is evaporated under reduced pressure, and the crude product is recrystallized.

Example 1

(±)-5-(4-Aminophenyl)-7,8-dihydro-8-methyl--7-/N-(4-morpholinoethyl)carbamoyl/-9H-- 1,3-dioxolo/4,5-h//2,3/-benzodiazepine

For the preparation see the general process above described using the appropriate nitro compound (see below).

Solvent for crystallization: dichloromethane.
M.p.: 262-264 °C.
Yield: 66 %.

| Analysis: for $C_{24}H_{29}N_5O_4$ (451.53) | | | |
|---|---|---|---|
| calculated | C 63.84 %, | H 6.47 %, | N 15.51 %; |
| found | C 63.96 %, | H 6.41 %, | N 15.30 %. |

$^1$H NMR /$(CD_3)_2SO$/ : δ 7.41 (2H, d, J=8.6 Hz), 6.98 (1H, s), 6.65 (2H, d, J=8.6 Hz), 6.54 (1H, s), 6.40 (1H, t, J=5.3 Hz), 6.06 (1H, s), 6.03 (1H, s), 5.50 (2H, broad s), 4.87 (1H, m), 3.64 (4H, m), 3.22 (2H, m), 2.83 (1H, dd, J=13.8 and 5.2 Hz), 2.42 (7H, m), 1.10 (3H, d, J=6.2 Hz).

**The starting compound**

(±)-7,8-Dihydro-8-methyl-7-/N-(4-morpholinoethyl)carbamoyl/-5-(4-nitrophenyl)-9H-1,3--dioxolo/4,5-h//2,3/benzodiazepine has been prepared as follows.

2.09 g (5.0 mmoles) of the imidazolide derivative (±)-7,8-Dihydro-8-methyl-5-(4-nitrophenyl)-9H--1,3-dioxolo/4,5-h//2,3/benzodiazepine-7--carboxylic acid-imidazolide prepared as described below are suspended in 100 $cm^3$ of dichloromethane, and, to the suspension, 1.44 g (1.44 $cm^3$, 11.0 mmoles) of (4-morpholinoethyl)amine are added. The reaction mixture is boiled for 10 hours, then washed three times using 30 $cm^3$ of water each time, dried over anhydrous magnesium sulfate, and evaporated under reduced pressure. The crude product is crystallized from 85 $cm^3$ of acetonitrile, the crystals are washed with 10 $cm^3$ of diethyl ether.

Thus, 1.83 g (76 %) of (±)-7,8-Dihydro-8-methyl-7-/N-(4-morpholinoethyl)carbamoyl/-5-(4-nitrophenyl)-9H-1,3--dioxolo/4,5-h//2,3/benzodiazepine
are obtained. M.p.: 198-203 °C.
$^1$H NMR ($CDCl_3$): δ 8.24 (2H, d, J=8.9 Hz), 7.68 (2H, d, J=8.9 Hz), 7.07 (1H, t, J=5.0 Hz), 6.73 (1H, s), 6.47 (1H, s), 6.01 (1H, s), 6.01 (1H, s), 6.00 (1H, s), 5.45 (1H, m), 3.71 (4H, m), 3.42 (2H, m), 3.12 (1H, dd, J=14.6 and 2.1 Hz), 2.87 (1H, dd, J=14.7 and 6.6 Hz), 2.55 (2H, m), 2.49 (4H, m), 0.97 (3H, d, J=6.6 Hz).

The (±)-7,8-Dihydro-8-methyl-5-(4-nitrophenyl)-9H--1,3-dioxolo/4,5-h//2,3/benzodiazepine-7-- carboxylic acid-imidazolide used above as starting substance has been prepared as follows.

3.25 g (10.0 mmoles) of (±)-7,8-dihydro--8-methyl-5-(4-nitrophenyl)-9H-1,3-dioxolo/4,5-h//2,3/benzodiazepine and 1.95 g (12.0 mmoles) of 1,1'-carbonyldiimidazole are boiled in 75 $cm^3$ of anhydrous tetrahydrofuran for 20 hours. The reaction mixture is cooled with ice-water, the product precipitated is filtered, and washed with 50 $cm^3$ of diethyl ether.

Thus, 3.58 g (85 %) of (±)-7,8-Dihydro-8-methyl-5-(4-nitrophenyl)-9H--1,3-dioxolo/4,5-h//2,3/benzodiazepine-7--carboxylicacid-imidazolide
are obtained. M.p.: 244-248 °C.

$^1H$ NMR ($CDCl_3$): δ 8.26 (2H, d, J=9.0 Hz), 7.91 (1H, s), 7.75 (2H, d, J=9.0 Hz), 7.31 (1H, s), 7.04 (1H, s), 6.88 (1H, s), 6.53 (1H, s), 6.08 (1H, d, J=1.3 Hz), 6.05 (1H, d, J=1.3 Hz), 5.24 (1H, m), 2.99 (1H, dd, J=14.5 and 4.8 Hz), 2.78 (1H, dd, J=14.6 and 10.2 Hz), 1.40 (3H, d, J=6.4 Hz).

Example 2

(±)-5-(4-Aminophenyl)-7-(N-cyclopropylcarbamoyl)-7,8-dihydro-8-methyl-9H-1,3--dioxolo/4,5-h//2,3/benzodiazepine

For the preparation see the general process above described using the appropriate nitro compound (see below).
Solvent for crystallization: ethanol.
M.p.: 158-160 °C.
Yield: 72 %.

| Analysis: for $C_{21}H_{22}N_4O_3$ (378.43) | | | |
|---|---|---|---|
| calculated | C 66.65 %, | H 5.85 %, | N 14.80 %; |
| found | C 65.96 %, | H 6.09 %, | N 14.52 %. |

$^1H$ NMR /$(CD_3)_2SO$/: δ 7.38 (2H, d, J=8.4 Hz), 6.98 (1H, s), 6.57 (2H, d, J=8.4 Hz), 6.53 (1H, s), 6.13 (1H, d, J=3.0 Hz), 6.06 (1H, s), 6.02 (3H, s), 5.68 (2H, broad s), 4.80 (1H, m), 2.78 (1H, dd, J=13.5 and 5.6 Hz), 2.50 (1H, m), 2.35 (1H, t, J=12.7 Hz), 1.07 (3H, d, J=6.1 Hz), 0.55 (2H, m), 0.45 (2H, m).

The starting compound (±)-7-(N-Cyclopropylcarbamoyl)-7,8-dihydro-8--methyl-5-(4-nitrophenyl)-9H-1,3-dioxolo/4,5-h//2,3/benzodiazepine has been prepared as follows.

2.09 g (5.0 mmoles) of the imidazolide derivative (±)-7,8-Dihydro-8-methyl-5-(4-nitrophenyl)-9H--1,3-dioxolo/4,5-h//2,3/benzodiazepine-7--carboxylicacid-imidazolide prepared as described at the end of Example 1 are boiled in 30 $cm^3$ of cyclopropylamine for 4 hours, then the amine is distilled off under reduced pressure. The residue is taken up in 75 $cm^3$ of dichloromethane, washed three times using 30 $cm^3$ of water each time, dried over anhydrous magnesium sulfate, and evaporated under reduced pressure. The crude product is crystallized from 50 $cm^3$ of ethanol, and washed with 10 $cm^3$ of diethyl ether.

Thus, 1.59 g (78 %) of (±)-7-(N-Cyclopropylcarbamoyl)-7,8-aihydro-8--methyl-5-(4-nitrophenyl)-9H-1,3-dioxolo/4,5-h//2,3/benzodiazepine
are obtained. M.p.: 198-203 °C.
$^1H$ NMR /$(CD_3)_2SO$/: δ 8.23 (2H, d, J=8.8 Hz), 7.77 (2H, d, J=8.( Hz), 6.99 (1H, s), 6.85 (1H, d, J=2.8 Hz), 6.48 (1H, s), 6.07 (2H, s), 5.20 (1H, m), 3.00 (1H, dd, J=14.5 and 2.1 Hz), 2.86 (1H, dd, J=14.5 and 7.2 Hz), 2.60 (1H, m), 0.90 (3H, d, J=6.4 Hz), 0.63 (2H, m), 0.53 (2H, m).

Example 3

(±)-5-(4-Aminophenyl)-7,8-dihydro-8-methyl--7-(N-methoxycarbamoyl)-9H-1,3-dioxolo/4,5-h//2,3/benzodiazepine

For the preparation see the general process above described using the appropriate nitro compound (see below).
Solvent for crystallization: ethanol.
M.p.: 159-162 °C.
Yield: 75 %.

| Analysis: for $C_{19}H_{20}N_4O_4$ (368.40) | | | |
|---|---|---|---|
| calculated: | C 61.95 %, | H 5.47 %, | N 15.21 %; |
| found: | C 61.62 %, | H 5.56 %, | N 15.32 %. |

$^1H$ NMR ($CDCl_3$): δ 9.23 (1H, s), 7.46 (2H, d, J=8.7 Hz), 6.99 (1H, s), 6.56 (2H, d, J=8.7 Hz), 6.53 (1H, s), 6.07 (1H, d, J=1.0 Hz), 6.03 (1H, d, J=1.0 Hz), 5.68 (2H, broad s), 4.75 (1H, m), 3.53 (3H, s), 2.79 (1H, dd, J=13.7 and 5.7 Hz), 2.36 (1H, dd, J=13.5 and 12.0 Hz), 1.12 (3H, d, J=6.1 Hz).

The starting compound (±)-7,8-Dihydro-8-methyl-7-(N-methoxycarbamoyl)-5-(4-nitrophenyl)-9H-1,3-dioxolo/4,5-h//2,3/benzodiazepine has been prepared as follows.

2.03 g (25.0 mmoles) of methoxyamine hydrochloride and 3.45 g (25.0 mmoles) of potassium carbonate are

stirred in 75 $cm^3$ of anhydrous dimethylformamide for half an hour, then, 2.09 g (5.0 mmoles) of the imidazolide derivative

(±)-7,8-Dihydro-8-methyl-5-(4-nitrophenyl)-9H--1,3-dioxolo/4,5-h//2,3/benzodiazepine-7--carboxylicacid-imidazolide prepared as described at the end of Example 1 are added. The reaction mixture is stirred for 6 hours, then the solvent is evaporated at a pressure of 55 Pa. The residue is suspended in 100 $cm^3$ of water, stirred for half an hour, filtered, washed with 50 $cm^3$ of water, and dried. The crude product is recrystallized from 35 $cm^3$ of tetrahydrofuran, and washed with 10 $cm^3$ of diethylether.

Thus, 2.30 g (68 %) of (±)-7,8-Dihydro-8-methyl-7-(N-methoxycarbamoyl)-5-(4-nitrophenyl)-9H-1,3-dioxolo/4,5-h//2,3/benzodiazepine
are obtained. M.p.: 156-162 °C.
$^1$H NMR /$(CD_3)_2SO$/: δ 10.00 (1H, s), 8.24 (2H, d, J=8.8 Hz), 7.90 (2H, d, J=8.8 Hz), 7.03 (1H, s), 6.51 (1H, s), 6.09 (1H, s), 6.08 (1H, s), 5.08 (1H, m), 3.63 (3H, s), 3.02 (1H, dd, J=14.4 and 3.5 Hz), 2.81 (1H, dd, J=14.4 and 8.2 Hz), 0.99 (3H, d, J=6.4 Hz).

Example 4

(±)-5-(4-Aminophenyl)-7-(N-aminocarbamoyl)--7,8-dihydro-8-methyl-9H-1,3-dioxolo/4,5-h//2,3/benzodiazepine

For the preparation see the general process above described using the appropriate nitro compound (see below).
Solvent for crystallization: acetonitrile.
M.p.: 160-170 °C.
Yield: 64 %.

| Analysis: for $C_{18}H_{19}N_5O_3$ (353.38) | | | |
|---|---|---|---|
| calculated | C 61.18 %, | H 5.42 %, | N 19.82 %; |
| found | C 59.68 %, | H 5.37 %, | N 19.32 %. |

$^1$H NMR ($CDCl_3$): δ 7.42 (2H, d, J=8.6 Hz), 7.07 (1H, s), 6.99 (1H, s), 6.56 (2H, d, J=8.6 Hz), 6.53 (1H, s), 6.07 (1H, d, J=0.8 Hz), 6.03 (1H, d, J=0.8 Hz), 5.68 (2H, s), 4.78 (1H, m), 3.96 (2H, s), 2.78 (1H, dd, J=13.7 and 5.7 Hz), 2.37 (1H, t, J=12.2 Hz), 1.11 (3H, d, J=6.2 Hz).

The starting compound (±)-7-(N-Aminocarbamoyl)-7,8-dihydro-8-methyl-5-(4-nitrophenyl)-9H-1,3-dioxolo/4,5-h//2,3/benzodiazepine has been prepared as follows.

2.09 g (5.0 mmoles) of the imidazolide derivative
(±)-7,8-Dihydro-8-methyl-5-(4-nitrophenyl)-9H--1,3-dioxolo/4,5-h//2,3/benzodiazepine-7--carboxylicacid-imidazolide
prepared as described at the end of Example 1 are suspended in 75 $cm^3$ of dichloromethane. To the suspension, 1.25 g (1.21 $cm^3$, 25.0 mmoles) of 98-100 % hydrazine hydrate are added. The reaction mixture is stirred at room temperature for 10 hours, then washed three times using 30 $cm^3$ of water each time, dried over anhydrous magnesium sulfate, and evaporated under reduced pressure. The crude product is crystallized from 45 $cm^3$ of ethanol, and the crystals are washed with 10 $cm^3$ of diethyl ether.

Thus, 1.04 g (54 %) of the title compound are obtained. M.p.: 219-220 °C.
$^1$H NMR ($CDCl_3$): δ 8.23 (2H, d, J=9.0 Hz), 7.62 (2H, d, J=9.0 Hz), 7.52 (1H, broad s), 6.73 (1H, s), 6.45 (1H, s), 6.01 (1H, d, J=1.3 Hz), 6.00 (1H, d, J=1.3 Hz), 5.38 (1H, m), 3.82 (2H, broad s), 3.12 (1H, dd, J=14.8 and 2.0 Hz), 2.86 (1H, dd, J=14.8 and 6.5 Hz), 0.99 (3H, d, J=6.6 Hz).

Example 5

(±)-5-(4-Aminophenyl)-7,8-dihydro-8-methyl--7-morpholinoacetyl-9H-1,3-dioxolo/4,5-h//2,3/benzodiazepine

5.00 g (11.0 mmoles) of (±)-7,8-Dihydro--8-methyl-7-morpholinoacetyl-5-(4-nitrophenyl)--9H-1,3-dioxolo/4,5-h//2,3/benzodiazepine prepared as described below are dissolved in 50 $cm^3$ of ethanol. To the solution, 0.50 g of 10 % palladium/carbon catalyst suspended in 1.0 $cm^3$ of water are added. Then, to the reaction mixture, a solution of 4.00 g (47.6 mmoles) of potassium formate in 4.0 $cm^3$ of water are added, drop by drop. The reaction mixture is stirred at room temperature for 2 hours, then again a solution of 2.00 g (23.8 mmoles) of potassium formate in 2.0 $cm^3$ of water are added, drop by drop. After further 2 hours' stirring, the catalyst is filtered, washed with a large quantity of ethanol, the solvent is evaporated under reduced pressure, and the residue is suspended in 100 $cm^3$ of diethyl ether. The crystals obtained are filtered, and the crude product is recrystallized from a mixture of acetonitrile and water.

Thus, 3.00 g (65 %) of the title compound are obtained. M.p.: 254-256 °C.

| Analysis: for $C_{23}H_{26}N_4O_4$ (422.49) | | |
|---|---|---|
| calculated | N 13.26 %, | H 6.20 %; |
| found | N 13.12 %, | H 6.48 %. |

$^1$H NMR ($CDCl_3$): δ 7.49 (2H, d, J=8.6 Hz), 6.75 (1H, s), 6.68 (2H, d, J=8.6 Hz), 6.58 (1H, s), 6.00 (1H, s), 5.97 (1H, s), 5.19 (1H, m), 4.1 (2H, bs), 3.69 (4H, t, J=4.6 Hz), 3.36 (1H, d, J=15.8 Hz), 3.07 (1H, d, J=15.8 Hz), 2.64 (2H, m), 2.53 (4H, m), 1.30 (3H, d, J=6.4 Hz).

The starting compound (±)-7,8-Dihydro-8-methyl-7-morpholinoacetyl--5-(4-nitrophenyl)-9H-1,3-dioxolo/4,5-h//2,3/benzodiazepine has been prepared as follows.

A mixture of 6.00 g (15.0 mmoles) of (+)-7,8-dihydro-7-(2-chloroacetyl)-8-methyl-- 5-(4-nitrophenyl)-9H-1,3-dioxolo/4,5-h//2,3/benzodiazepine, 3.00 g (36.0 mmoles) of morpholine and 30 $cm^3$ of acetonitrile is boiled for 2 hours. Then, the reaction mixture is evaporated. To the evaporation residue, 100 $cm^3$ of diethyl ether are added, the crystals obtained are filtered, and recrystallized from a mixture of 2-propanol and water.

Thus, 4.90 g (73 %) of the title compound are obtained. M.p. 206-208 °C.

Example 6

5-(4-Aminopheayl)-8-methyl-7H-1,3-dioxolo/4,5-h//2,3/benzodiazepine-7-carboxylic acid-(2-morpholino-4-ylethyl) amide

2.0 g (4.17 mmoles) of 8-Methyl-5-(4-nitrophenyl)-7H-1,3-dioxolo/4,5-h//2,3/benzodiazepine-7-carboxylic acid-(2-morpholino-4-ylethyl)amide prepared as described below are transferred into a mixture of 80 $cm^3$ of ethanol and 20 $cm^3$ of water. To the mixture, 0.4 g of 10 % palladium/carbon catalyst, then, in 4 minutes, 4.0 $cm^3$ (80.6 mmoles) of 98 % hydrazine hydrate are added at 15 to 20 °C. The mixture is stirred at 25 °C for 4.5 hours, the catalyst is filtered, and washed with ethanol. The filtrate is evaporated under reduced pressure, and, to the residue, 120 $cm^3$ of water are added. After an hour's stirring, the crystals are filtered, and washed with water. The crude product is transferred to a silica gel column that is eluted with a mixture of chloroform and methanol. The adequate fraction is evaporated, and the evaporation residue is crystallized from diethyl ether. The crystals obtained are filtered, and washed with diethyl ether.

Thus, 0.52 g (27.8 %) of the title compound are obtained. M.p.: 249-251 °C.

| Analysis: for $C_{24}H_{27}N_5O_4$ (449.51) | | | |
|---|---|---|---|
| calculated | C 64.13 %, | H 6.05 %, | N 15.58 %; |
| found | C 64.36 %, | H 6.20 %, | N 15.20 %. |

$^1$H NMR ($CDCl_3$): δ 7.36 (2H, d, J=8.3 Hz), 6.79 (1H, m), 6.67 (2H, s), 6.65 (2H, d, J=8.3 Hz), 6.13 (1H, s), 6.01 (1H, s), 5.95 (1H, s), 4.01 (2H, bs), 3.80 (4H, t, J=4.5 Hz), 3.5-3.3 (2H, m), 2.65-2.4 (6H, m), 2.23 (3H, s).

The starting compound 8-Methyl-5-(4-nitrophenyl)-7H-1,3-dioxolo/4,5-h//2,3/benzodiazepine-7-carboxylic acid-(2-morpholino-4-ylethyl)amide has been prepared as follows.

10.0 g (22.6 mmoles) of Phenyl 8-methyl-5-(4-nitrophenyl)-7H-1,3--dioxolo/4,5-h//2,3/benzodiazepine-7--carboxylate prepared as described below, 100 $cm^3$ of ethanol and 19.08 g (146.6 mmoles) of 4-(2-aminoethyl)morpholine are transferred to an acidresistant steel bomb tube of 200 $cm^3$ capacity. The bomb tube is sealed, and the mixture is stirred at 110 °C for 24 hours. On the next day, the bomb tube is opened, and the mixture is evaporated under reduced pressure. The evaporation residue is stirred in 400 $cm^3$ of water for 5 hours, then extracted three times using 200 $cm^3$ of chloroform each time. The organic phase is dried over anhydrous magnesium sulfate, and evaporated under reduced pressure. The 8.0 g of evaporation residue are transferred to a silica gel column that is eluted with a mixture of chloroform and methanol. The adequate fraction is evaporated, the evaporation residue is stirred in 50 $cm^3$ of diisopropyl ether for an hour. The crystals are filtered, and washed with diisopropyl ether.

Thus, 5.8 g (35.8 %) of the title compound are obtained. M.p.: 218-220 °C.
$^1$H NMR (DMSO-$d_6$): δ 8.27 (2H, d, J=9.0 Hz), 7.88 (2H, d, J=9.0 Hz), 7.06 (1H, t, J=2.8 Hz), 6.98 (1H, s), 6.59 (1H, s), 6.31 (1H, s), 6.12 (2H, s), 3.60 (4H, m); 3.3 (2H, s), 2.5-2.1 (6H, m), 2.09 (3H, s).

The Phenyl 8-methyl-5-(4-nitrophenyl)-7H-1,3--dioxolo/4,5-h//2,3/benzodiazepine-7--carboxylate used above as starting substance has been prepared as follows.

20.0 g (61.9 mmoles) of 8-methyl-5-(4--nitrophenyl)-7H-1,3-dioxolo/4,5-h//2,3/benzodiazepine are added to

600 $cm^3$ of chloroform, and, to the mixture, 37. 2 g (237.6 mmoles) of phenyl chloroformate are added, drop by drop, at 5 to 10 °C in 15 minutes. The suspension is boiled for 7 hours, while the mixture becomes a clear solution. After cooling, the solution is evaporated under reduced pressure, to the evaporation residue, 300 $cm^3$ of diethyl ether are added, and the mixture is stirred at 25 °C for 16 hours. The crystals obtained are filtered, and washed three times using 50 $cm^3$ of diethyl ether each time.

Thus, 26.0 g (94.9 %) of Phenyl 8-methyl-5-(4-nitrophenyl)-7H-1,3--dioxolo/4,5-h//2,3/benzodiazepine-7--carboxylate

are obtained. M.p.: 218-220 °C.

$^1H$ NMR ($CDCl_3$): δ 8.25 (2H; d, J=9.0 Hz), 7.77 (2H, d, J=9.0 Hz), 7.4 (2H, m), 7.2 (3H, m), 6.81 (1H, s), 6.55 (1H, s), 6.07 (1H, s), 6.02 (1H, s), 6.36 (1H, qa, J=1.1 Hz), 2.36 (3H, d, J=1.1 Hz).

Example 7

5-(4-Aminophenyl)-8-methyl-7-/2-(2-morpholinoethylamino)acetyl/-7H-1,3-dioxolo/4,5-h//2,3/benzodiazepine monohydrate

6.0 g (10.3 mmoles) of 7-[2-/N-Benzyl-(2-morpholinoethyl)amino/acetyl]-8-methyl-5-(4-nitrophenyl)-7H--1,3-dioxolo/4,5-h//2,3/benzodiazepine prepared as described below are transferred into a mixture of 240 $cm^3$ of methanol and 50 $cm^3$ of water. To the mixture, 4.8 g of 10 % palladium/carbon catalyst, then, in 20 minutes, 24.0 $cm^3$ (484 mmoles) of 98 % hydrazine hydrate are added at 20 to 25 °C. The mixture is stirred at 25 °C for 100 hours, then further 2.4 g of 10 % palladium/carbon catalyst and 12.0 $cm^3$ (242 mmoles) of 98 % hydrazine hydrate are added. After further 72 hours' stirring, the catalyst is filtered, washed with methanol, and the filtrate is evaporated under reduced pressure. To the residue, 100 $cm^3$ of water and 150 $cm^3$ of dichloromethane are added. After 5 minutes' stirring, the phases are separated, the aqueous phase is still extracted twice with 150 $cm^3$ of dichloromethane each time. The organic phase is dried, and evaporated under reduced pressure. The evaporation residue is transferred to a silica gel column that is eluted with a mixture of chloroform and methanol. The adequate fraction is evaporated, and the evaporation residue is crystallized from diisopropyl ether. The crystals obtained are filtered, and washed with diisopropyl ether.

Thus, 3.65 g (76.7 %) of the title compound are obtained. M.p.: 92-94 °C.

| Analysis: for $C_{25}H_{29}N_5O_4.H_2O$ (481.56) | |
|---|---|
| calculated | N 14.54 %; |
| found | N 14.25 %. |

$^1H$ NMR (DMSO-$d_6$): δ 7.18 (2H, d, J=8.4 Hz), 7.00 (1H, s), 6.72 (1H, s), 6.58 (2H, d, J=8.4 Hz), 6.48 (1H, s), 6.15 (1H, s), 6.08 (1H, s), 5.75 (2H, bs), 3.73 (1H, d, J=16.9 Hz), 3.54 (4H, t, J=4.6 Hz), 3.30 (1H, d, J=16.9 Hz), 3.05 (1H, m), 2.62 (2H, t, J=6.0 Hz), 2.40-2.25 (6H, m), 2.16 (3H, s).

The starting compound 7-[2-/N-Benzyl-(2-morpholinoethyl)amino/acetyl ]-8-methyl-5-(4-nitrophenyl)-7H--1,3-dioxolo/4,5-h//2,3/benzodiazepine has been prepared as follows.

A mixture of 12.0 g (30 mmoles) of 7-(2-Chloroacetyl)-8-methyl-5-(4-nitrophenyl)--7H-1,3-dioxolo/4,5-h//2,3/benzodiazepine prepared as described below,

250 $cm^3$ of acetonitrile and 14.9 g (66 mmoles) of benzyl-(2-morpholine-4-ylethyl)amine is boiled for 7 hours. After cooling, the reaction mixture is filtered, and the filtrate is evaporated under reduced pressure. The evaporation residue is dissolved in 300 $cm^3$ of dichloromethane, washed twice with 100 $cm^3$ of water each time, and the organic phase is evaporated under reduced pressure. The evaporation residue (11.4 g) is transferred to a silica gel column that is eluted with a mixture of chloroform and methanol. The adequate fraction is evaporated under reduced pressure, then treated at a pressure of 0.1 mm Hg.

Thus, 10.0 g (57.1 %) of crystalline foam are obtained. M.p.: 69-70 °C.

| Analysis: for $C_{32}H_{33}N_5O_6$ (583.65) | |
|---|---|
| calculated | N 12.00 %; |
| found | N 11.82 %. |

$^1H$ NMR ($CDCl_3$): δ 8.23 (2H, d, J=8.8 Hz), 7.59 (2H, d, J=8.8 Hz), 7.25 (5H, m), 6.77 (1H, s), 6.44 (1H, s), 6.33 (1H, s), 6.04 (2H, s), 3.91 (3H, bs), 3.62 (5H, m), 2.93 (2H, m), 2.48 (2H, m), 2.37 (4H, m), 2.28 (3H, s).

The 8-methyl-5-(4-nitrophenyl)-7H-1,3--dioxolo/4,5-h//2,3/benzodiazepine above used as starting substance has been prepared as follows.

To 45 $cm^3$ (564.6 mmoles) of chloroacetyl chloride, 15.0 g (46.4 mmoles) of 8-methyl--5-(4-nitrophenyl)-7H-1,3-dioxolo/4,5-h//2,3/benzodiazepine are added under ice-water cooling in 10 minutes. After 5 minutes' stirring at 25 °C, the solution becomes cloudy. The mixture is stirred at 80 °C for 60 minutes, then boiled for 15 minutes. After cooling, the mixture is poured onto 450 g of ice, stirred for 3 hours, the crystals precipitated are filtered, washed three times using 60 $cm^3$ of water each time, and dried under a lamp emitting infra red radiation. The crude product is boiled in 150 $cm^3$ of ethanol for 5 minutes. After cooling, the crystals are filtered, washed with ethanol and diethyl ether.

Thus, 15.5 g (83.5 %) of 7-(2-Chloroacetyl)-8-methyl-5-(4-nitrophenyl)--7H-1,3-dioxolo/4,5-h//2,3/benzodiazepine are obtained. M.p.: 228-229 °C.

| Analysis: for $C_{19}H_{14}ClN_3O_5$ (399.79) | |
|---|---|
| calculated | N 10.51 %; |
| found | N 10.28 %. |

$^1H$ NMR ($CDCl_3$): δ 8.28 (2H, d, J=8.8 Hz), 7.71 (2H, d, J=8.8 Hz), 6.77 (1H, s), 6.48 (1H, s), 6.38 (1H, bs), 6.05 (2H, s), 4.09 (2H, s), 2.28 (3H, s).

Example 8

5-(4-Aminophenyl)-8-methyl-7-/3-(2-morpholinoethylamino)propionyl/-7H-1,3-dioxolo/4,5-h//2,3/benzodiazepine

The 7-[3-/N-Benzyl-(2-morpholinoethyl)amino/propionyl ]-8-methyl-5-(4-nitrophenyl)-7H--1,3-dioxolo/4,5-h//2,3/- benzodiazepine prepared as described below is reduced as follows.

5.2 g (8.7 mmoles) of 7-[3-/N-Benzyl-(2-morpholinoethyl)amino/propionyl]-8-methyl-5-(4-nitrophenyl)-7H--1,3-dioxolo/4,5-h//2,3/- benzodiazepine are added to a mixture of 175 $cm^3$ of methanol and 35 $cm^3$ of water. To the mixture, 1.4 g of 10 palladium/carbon catalyst, and, in 10 minutes, 7.0 $cm^3$ (141 mmoles) of 98 % hydrazine hydrate are added at 20 to 25 °C. The mixture is stirred at 25 °C f or 24 hours. Then, the catalyst is filtered, washed with methanol, and the filtrate is evaporated under reduced pressure. To the residue, 100 $cm^3$ of water and 150 $cm^3$ of dichloromethane are added. After 5 minutes' stirring, the phases are separated, and the aqueous phase is still twice extracted with 150 $cm^3$ of dichloromethane each time. The organic phase is dried, evaporated under reduced pressure, and the evaporation residue is transferred to a silica gel column that is eluted with a mixture of chloroform and methanol. The appropriate fraction is evaporated, and the evaporation residue is crystallized from diisopropyl ether. The crystals obtained are filtered, and washed with diisopropyl ether.

Thus, 0.4 g (8.2 %) of 5-(4-Aminophenyl)-7-[3-/N-benzyl-(2--morpholinoethylamino)/propionyl]-8-methyl--7H-1,3-dioxolo/4,5-h//2,3/benzodiazepine are obtained (thin-layer chromatography: using a mixture of ethanol and ammonia in a ratio of 9:1, $R_f$ = 0.75).
M.p.: 114-116 °C.
$^1H$ NMR ($CDCl_3$): δ 7.31 (2H, d, J=8.7 Hz), 7.26 (5H, m), 6.72 (1H, s), 6.64 (2H, d, J=8.7 Hz), 6.62 (1H, s), 6.31 (1H, d, J=1.6 Hz), 6.05 (1H, d, J=1.6 Hz), 5.97 (1H, d, J=1.6 Hz), 3 .98 (2H, s), 3.64 (6H, m), 2.93-2.68 (4H, m), 2.63 (2H, m), 2.44 (2H, m), 2.36 (4H, m), 2.25 (3H, s).

Together with the latter also the debenzyl derivative of 5-(4-Aminophenyl)-7-[3-/N-benzyl-(2--morpholinoethylamino)/propionyl]-8-methyl--7H-1,3-dioxolo/4,5-h//2,3/benzodiazepine is formed in the reaction. The two compounds are separated by the above column chromatographic method. The appropriate fraction is evaporated, and the evaporation residue is crystallized from diisopropyl ether. The crystals obtained are filtered, and washed with diisopropyl ether.

Thus, 0.7 g (16.9 %) of 5-(4-Aminophenyl)-8-methyl-7-/3-(2-morpholinoethylamino)propionyl/-7H-1,3-dioxolo/4,5-h//2,3/benzodiazepine are obtained (thin-layer chromatography: using a mixture of ethanol and ammonia in a ratio of 9:1, $R_f$ = 0.65).
M.p.: 122-124 °C.

| Analysis: for $C_{26}H_{31}N_5O_4$ (477.57) | |
|---|---|
| calculated | N 14.66 %; |
| found | N 14.46 %. |

$^1H$ NMR ($CDCl_3$): δ 7.32 (2H, d, J=8.6 Hz), 6.67 (2H, s), 6.64 (2H, d, J=8.6 Hz), 6.32 (1H, d, J=1.1 Hz), 6.04 (1H, d, J=1.1 Hz), 5.97 (1H, d, J=1.1 Hz), 4.10 (2H, bs), 3.68 (4H, t, J=4.7 Hz), 3.2-2.5 (8H, m), 2.43 (4H, t, J=4.6 Hz), 2.27 (3H, d, J=1.1 Hz).

The starting compound 7-[3-/N-Benzyl-(2-morpholinoethyl)amino/propionyl]-8-methyl-5-(4-nitrophenyl)-7H--1,3-dioxolo/4,5-h//2,3/- benzodiazepine has been prepared as follows.

A mixture of 10.34 g (25 mmoles) of 7-(3-Chloropropionyl)-8-methyl-5-(4-nitrophenyl)-7H-1,3-dioxolo/4,5-h//2,3/benzodiazepine prepared as described below, 250 $cm^3$ of acetonitrile and 12.42 g (55.0 mmoles) of benzyl-(2-morpholine-4-ylethyl)amine is boiled for 8 hours. The reaction mixture is evaporated under reduced pressure. The evaporation residue is crystallized from 150 $cm^3$ of water, stirred at 25 °C for 2 hours, the crystals obtained are filtered, and washed with water. The crude product (10.8 g) is transferred to a silica gel column that is eluted with a mixture of chloroform and methanol. The adequate fraction is evaporated under reduced pressure, and treated at a pressure of 0.1 mm Hg. The crystals are collected.

Thus, 9.2 g (61.7 %) of 7-[3-/N-Benzyl-(2-morpholinoethyl)amino/propionyl ]-8-methyl-5-(4-nitrophenyl)-7H--1,3-dioxolo/4,5-h//2,3/- benzodiazepine are obtained. M.p.: 74-75 °C.

| Analysis: for $C_{33}H_{35}N_5O_6$ (597.68) | | | |
|---|---|---|---|
| calculated | C 66.32 %, | H 5.90 %, | N 11.72 %; |
| found | C 65.85 %, | H 5.80 %, | N 11.78 %. |

$^1H$ NMR ($CDCl_3$): δ 8.23 (2H, d, J=8.7 Hz), 7.59 (2H, d, J=8.7 Hz), 7.25 (5H, m), 6.75 (1H, s), 6.39 (1H, s), 6.33 (1H, s), 6.02 (2H, s), 3.65 (6H, m), 3.00-2.40 (12H, m), 2.28 (3H, d, J=1.2 Hz).

The 7-(3-Chloropropionyl)-8-methyl-5-(4-nitrophenyl)-7H-1,3-dioxolo/4,5-h//2,3/benzodiazepine used above as starting substance has been prepared as follows.

To 45 $cm^3$ (461.9 mmoles) of 3-chloropropionyl chloride, 15.0 g (46.4 mmoles) of 8-methyl-5-(4-nitrophenyl)-7H-1,3-dioxolo/4,5-h//2,3/benzodiazepine are added under ice-water cooling in 10 minutes. The mixture is stirred at 25 °C for 22 hours, then poured onto 450 g of ice. After 3 hours' stirring, the crystals precipitated are filtered, washed three times with 60 $cm^3$ of water each time, and dried under a lamp emitting infra red radiation. The crude product is dissolved in 300 $cm^3$ of dichloromethane, and washed with 200 $cm^3$ of water. The organic phase is evaporated under reduced pressure, and the evaporation residue is boiled in 100 $cm^3$ of ethanol for 10 minutes. After cooling, the crystals are filtered, washed with ethanol and diethyl ether.

Thus, 14.1 g (73.4 %) of 7-(3-Chloropropionyl)-8-methyl-5-(4-nitrophenyl)-7H-1,3-dioxolo/4,5-h//2,3/benzodiazepine are obtained. M.p.: 207-209 °C.

| Analysis: for $C_{20}H_{16}ClN_3O_5$ (413.82) | | | | |
|---|---|---|---|---|
| calculated | C 58.05 %, | H 3.90 %, | N 10.15 %, | Cl 8.57 %; |
| found | C 58.66 %, | H 4.02 %, | N 9.96 %, | Cl 8.53 %. |

$^1H$ NMR ($CDCl_3$): δ 8 .28 (2H, d, J=8.8 Hz), 7.71 (2H, d, J=8.8 Hz), 6.77 (1H, s), 6.48 (1H, s), 6.35 (1H, bs), 6.05 (2H, bs), 3.86 (2H, m), 3.1-2.9 (2H, m), 2.27 (3H, s).

Example 9

5-(4-Aminophenyl)-7-(2-chloroacetyl)-8-methyl-7H-1,3-dioxolo/4,5-h//2,3/benzodiazepine

4.0 g (10 mmoles) of 7-(2-Chloroacetyl)-8-methyl-5-(4-nitrophenyl)-7H-1,3-dioxolo/4,5-h//2,3/benzodiazepine prepared as described at the end of Example 7 are transferred into 160 $cm^3$ of ethanol, 9.0 g (40 mmoles) of crystalline tin(II) chloride ($SnCl_2.2H_2O$) are added, and the mixture is boiled for 1.5 hours. After cooling, the reaction mixture is evaporated. To the residue, 120 $cm^3$ of water are added, and the mixture is extracted three times using 100 $cm^3$ of dichloromethane each time. The combined dichloromethane layers are washed twice with 30 $cm^3$ of 5 % aqueous sodium hydroxide solution each time, and twice with 150 $cm^3$ of water each time, then dried, and evaporated under reduced pressure. To the evaporation residue, 50 $cm^3$ of diisopropyl ether are added. After 30 minutes' stirring, the crystals are filtered.

Thus, 1.9 g (51.6 %) of 5-(4-Aminophenyl)-7-(2-chloroacetyl)-8-methyl-7H-1,3-dioxolo/4,5-h//2,3/benzodiazepine are obtained. M.p.: 197-199 °C.
$^1H$ NMR ($CDCl_3$ + DMSO-$d_6$): δ 7.27 (2H, d, J=8.6 Hz), 6.75 (1H, s), 6.72 (1H, s), 6.65 (2H, d, J=8.6 Hz), 6.35 (1H, s), 6.02 (2H, bs), 4.59 (2H, bs), 4.35 (2H, m), 2.25 (3H, d, J=1.0 Hz).

Example 10

5-(4-Aminophenyl)-7-(3-chloropropionyl)-8-- methyl-7H-1,3-dioxolo/4,5-h//2,3/benzodiazepine

6.18 g (15 mmoles) of 7-(3-Chloropropionyl)-8-methyl-5-(4-nitrophenyl)-7H-1,3-dioxolo/4,5-h//2,3/benzodiazepine prepared as described at the end of Example 8 are transferred into 180 $cm^3$ of ethanol, 16.92 g (75 mmoles) of crystalline tin(II) chloride ($SnCl_2.2H_2O$) are added, and the mixture is boiled for 70 minutes. After cooling, the reaction mixture is evaporated under reduced pressure. To the residue, 200 $cm^3$ of water are added, and the pH of the solution is adjusted to 11 by the addition of 10 % aqueous sodium hydroxide solution. The mixture is extracted five times using 200 $cm^3$ of dichloromethane each time. The combined dichloromethane layers are washed twice with 250 $cm^3$ of water each time, dried over anhydrous magnesium sulfate, and evaporated under reduced pressure. To the evaporation residue, 100 $cm^3$ of diisopropyl ether are added. After 60 minutes' stirring, the crystals are filtered, and washed with diisopropyl ether. The crude product is recrystallized from ethanol.

Thus, 1.75 g (30.7 %) of 5-(4-Aminophenyl)-7-(3-chloropropionyl)-8-- methyl-7H-1,3-dioxolo/4,5-h//2,3/benzodiazepine are obtained: M.p.: 162-165 °C.

| Analysis: for $C_{20}H_{18}ClN_3O_3$ (383.84) | |
|---|---|
| calculated | N 10.95 %; |
| found | N 10.65 %. |

$^1H$ NMR ($CDCl_3$): δ 7.33 (2H, d, J=8.7 Hz), 6.73 (2H, s), 6.66 (2H, d, J=8.7 Hz), 6.33 (1H, d, J=1.3 Hz), 6.05 (1H, d, J=1.3 Hz), 5.98 (3H, d, J=1.3 Hz), 4.02 (2H, bs), 3.85 (1H, m), 3.75 (1H, m), 2.90 (1H, m), 2.27 (3H, d, J=1.3 Hz).

Example 11

5-(4-Aminophenyl)-8-methyl-7-methylcarbamoyl-7H-1,3-dioxolo/4,5-h//2,3/benzodiazepine

4.0 g (10.5 mmoles) of 8-Methyl-7-methylcarbamoyl-5-(4-nitrophenyl)-7H-1,3-dioxolo-/4,5-h//2,3/benzodiazepine prepared as described below are transferred into 200 $cm^3$ of ethanol, 10.64 g (47.2 mmoles) of crystalline tin (II) chloride ($SnCl_2.2H_2O$) are added, and the mixture is boiled for 2 hours. After cooling, the reaction mixture is evaporated under reduced pressure. To the residue, 150 $cm^3$ of water are added, and the pH of the solution is adjusted to 11 by the addition of 10 % aqueous sodium hydroxide solution. The mixture is extracted three times using 300 $cm^3$ of dichloromethane each time. The combined dichloromethane layers are dried over anhydrous magnesium sulfate, and evaporated under reduced pressure. To the evaporation residue, 30 $cm^3$ of diisopropyl ether are added. After 60 minutes' stirring, the crystals are filtered, and washed with diisopropyl ether.

Thus, 1.02 g (27.7 %) of 5-(4-Aminophenyl)-8-methyl-7-methylcarbamoyl- 7H-1,3-dioxolo/4,5-h//2,3/benzodiazepine are obtained. M.p.: 188-190 °C.

$^1H$ NMR ($CDCl_3$): δ 7.27 (2H, d, J=8.6 Hz), 6.66 (1H, s), 6. 65 (1H, s), 6. 62 (2H, d, J=8.6 Hz), 6.13 (1H, d, J=1.0 Hz), 6.05 (1H, m), 6.00 (1H, s), 5.94 (1H, s), 3.7 (2H, bs), 2.92 (3H, d, J=5.0 Hz), 2.22 (3H, d, J=1.2 Hz).

The starting compound 8-Methyl-7-methylcarbamoyl-5-(4-nitrophenyl)-7H-1,3-dioxolo-/4,5-h//2,3/benzodiazepine has been prepared as follows.

5 g (11.3 mmoles) of Phenyl 8-methyl-5-(4-nitrophenyl)-7H-1,3-- dioxolo/4,5-h//2,3/benzodiazepine-7-- carboxylate prepared as described below, 50 $cm^3$ of ethanol and 14.4 $cm^3$ (136.6 mmoles) of 33 % methylamine in ethanol are transferred to an acid resistant steel bomb tube of 200 $cm^3$ capacity. The bomb tube is sealed, and the mixture is stirred at 90 °C for 8 hours. The mixture is allowed to stand at 25 °C for a night, on the other day the bomb tube is opened. The crystals precipitated are filtered, washed three times using 5 $cm^3$ of ethanol each time, then twice with 20 $cm^3$ of diethyl ether each time.

Thus, 3.6 g (83.9 %) of 8-Methyl-7-methylcarbamoyl-5-(4-nitrophenyl)-7H-1,3-dioxolo-/4,5-h//2,3/benzodiazepine are obtained. M.p.: higher than 250 °C.

$^1H$ NMR ($CDCl_3$): δ 8.25 (2H, d, J=8.8 Hz), 7.67 (2H, d, J=8.8 Hz), 6.70 (1H, s), 6.40 (1H, s), 6.15 (1H, s), 6.10 (1H, m), 6.01 (2H, s), 2.97 (3H, d, J=4.8 Hz), 2.21 (3H, s).

The Phenyl 8-methyl-5-(4-nitrophenyl)-7H-1,3-dioxolo/4,5-h//2,3/benaodiacenine-7-carboxylate used above as starting substance has been prepared as follows.

20.0 g (61.9 mmoles) of 8-methyl-5-(4-nitrophenyl)-7H-1,3-dioxolo/4,5-h//2,3/benzodiazepine are added to 600 $cm^3$ of chloroform, and, to the mixture, 37.2 g (237.6 mmoles) of phenyl chloroformate are added, drop by drop, at 5 to 10 °C in 15 minutes. The suspension is boiled for 7 hours, while the mixture becomes a clear solution. After cooling, the solution is evaporated under reduced pressure, to the evaporation residue, 300 $cm^3$ of diethyl ether are

added, and the mixture is stirred at 25 °C for 16 hours. The crystals obtained are filtered, and washed three times using 50 $cm^3$ of diethyl ether each time.

Thus, 26.0 g (94.9 %) of Phenyl 8-methyl-5-(4-nitrophenyl)-7H-1,3-dioxolo/4,5-h//2,3/benzodiazepine-7- carboxylate are obtained. M.p.: 218-220 °C.

$^1H$ NMR ($CDCl_3$): δ 8.25 (2H; d, J=9.0 Hz), 7.77 (2H, d, J=9.0 Hz), 7.4 (2H, m), 7.2 (3H, m), 6.81 (1H, s), 6.55 (1H, s), 6.07 (1H, s), 6.02 (1H, s), 6.36 (1H, ga, J=1.1 Hz), 2.36 (3H, d, J=1.1 Hz).

## Claims

**1.** A 1,3-dioxolo/4,5-h//2,3/benzodiazepine derivative of the formula I

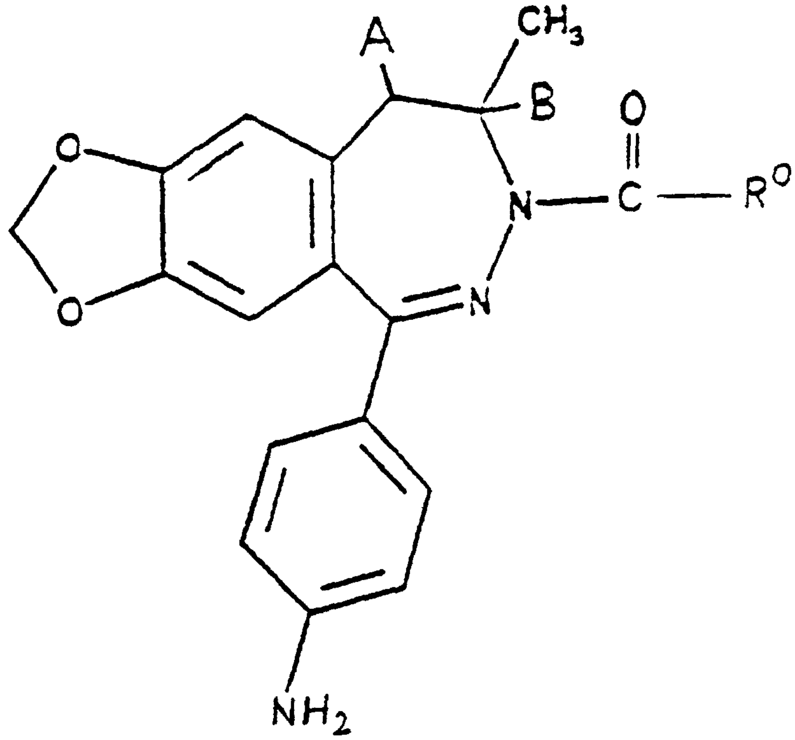

I ,

wherein

a) A and B represent hydrogen or together form a valence bond and

$R^0$ stands for a group of

formula $-(CH_2)_m-R$,

wherein

R represents a halogen atom or a group of formula $-NR^3R^4$, wherein

$R^3$ represents hydrogen and

$R^4$ means $C_{3-6}$ cycloalkyl, $C_{1-4}$ alkoxy, amino or $C_{1-4}$ alkyl, which latter is substituted by a saturated heterocyclic group having 5 or 6 members and comprising 1 nitrogen atom or 1 nitrogen atom and 1 oxygen atom as the heteroatom(s),

$R^3$ and $R^4$ mean, independently from each other, $C_{3-6}$ cycloalkyl, $C_{1-4}$ alkoxy, amino or $C_{1-4}$ alkyl, which latter is substituted by a saturated heterocyclic group having 5 or 6 members and comprising 1 nitrogen atom or 1 nitrogen atom and 1 oxygen atom as the heteroatom(s),
or

$R^3$ and $R^4$ together form with the adjacent nitrogen atom and optionally with an oxygen atom a saturated heterocyclic group having 5 or 6 members
and

m is 0, 1 or 2
or

b) A and B together form a valence bond,

$R^0$ stands for a group of formula $-(CH_2)_m-R$, wherein

R additionally to the meanings defined under a) alternatively represents a group of formula $-NR^3R^4$,
wherein

$R^3$ and $R^4$ mean, independently from each other, hydrogen or unsubstituted $C_{1-4}$ alkyl
and

m is 0
or

c) A and B represent hydrogen,

$R^0$ stands for a group of formula $-(CH_2)_m-R$, wherein

R additionally to the meanings defined under a) alternatively represents a group of formula $-NR^3R^4$, wherein

$R^3$ means a hydrogen atom and
$R^4$ stands for an unsubstituted $C_{2-4}$ alkyl group
and

m is 0,

and pharmaceutically suitable acid addition salts thereof.

**2.** A 1,3-dioxolo/4,5-h//2,3/benzodiazepine derivative according to claim 1, wherein

A and B represent hydrogen atoms
and the halogen atom which may be represented by R is a chloro atom
or the cycloalkyl group for which

$R^3$ and/or $R^4$ may stand, is/are [a] cyclopropyl group(s) and

m is as defined in claim 1,

and pharmaceutically suitable acid addition salts thereof.

**3.** A 1,3-dioxolo/4,5-h//2,3/benzodiazepine derivative according to claim 1, wherein

A and B represent hydrogen atoms,
the alkyl group(s) for which $R^3$ and/or $R^4$ may stand is/are [a] $C_{1-2}$ alkyl group(s)
and the heterocyclic group by which the alkyl group(s), for which $R^3$ and/or $R^4$ may stand, may be substituted or the heterocyclic group which may be formed by $R^3$ and/or $R^4$ with the adjacent nitrogen atom and with an oxygen atom is a morpholino group
and
m has a value of 0 or 1,

and pharmaceutically suitable acid addition salts thereof.

**4.** A 1,3-dioxolo/4,5-h//2,3/benzodiazepine derivative according to claim 3, wherein

A and B represent hydrogen atoms,
$R^3$ represents a hydrogen atom,
$R^4$ stands for a cyclopropyl group or an amino group
and
m has a value of 0

and pharmaceutically suitable acid addition salts thereof.

**5.** A 8-methyl-7H-1,3-dioxolo/4,5-h//2,3/benzodiazepine derivative according to claim 1, wherein

A and B together form a valence bond,
and
the halogen atom which may be represented by R is a chloro atom
or
the alkyl group(s) for which
the heterocyclic group(s), by which the alkyl group(s), for which $R^3$ and/or $R^4$ may stand, may be substituted, is/are [a] morpholino group(s)
and
m has a value of 0, 1 or 2,

and pharmaceutically suitable acid addition salts thereof.

**6.** A process for the preparation of a 1,3-dioxolo/4,5-h//2,3/benzodiazepine derivative of the formula I, wherein A, B and $R^0$ are as defined in claim 1, according to claim 1, and pharmaceutically suitable acid addition salts thereof, **characterized in that**

a) for the preparation of a compound of the formula I, wherein $R^0$ represents a group of the formula $-(CH_2)_m-R$, in which R stands for a halogen atom, m has a value of 0, 1 or 2 and A and B represent hydrogen atoms, the 7,8-dihydro-8-methyl-5-(4-nitrophenyl)-9H-1,3-dioxolo/4,5-h//2,3/benzodiazepine of the formula III

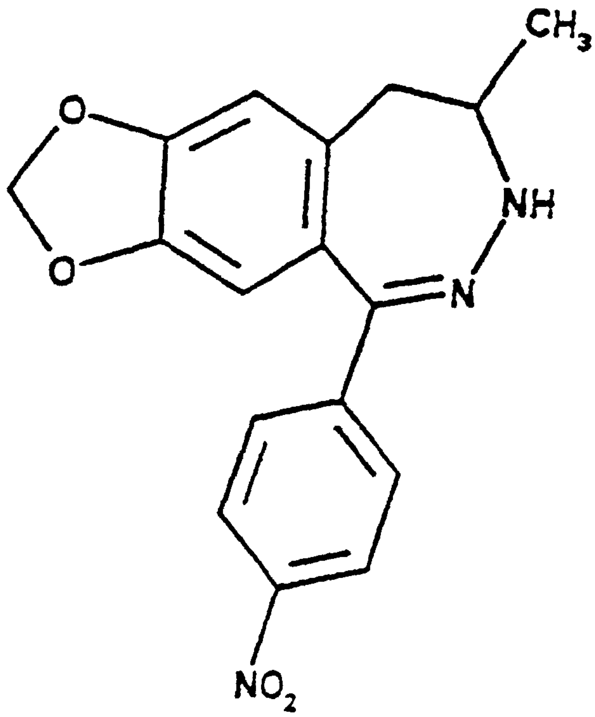

III

is reacted with a reagent of the formula VI

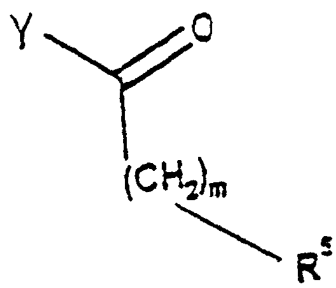

VI ,

wherein Y represents a leaving group and $R^5$ is a halogen atom and the obtained compound in which instead of the amino group on the phenyl ring of the formula I there is a nitro group and $R^0$, A and B are as defined in connection with the formula I, is transformed into a compound of the formula I by reduction;

or

b) for the preparation of a compound of the formula I, wherein $R^0$ represents a group of the formula $-(CH_2)_m$-R, wherein R stands for a group of the formula $-NR^3R^4$, in which $R^3$, $R^4$ and m are as defined in connection with formula I and A and B represent hydrogen atoms, the 7,8-dihydro-8- methyl-5-(4-nitrophenyl)-9H-2,3-dioxolo/4,5- h//2,3/benzodiazepine of the formula III is reacted with a reagent of the formula VI, wherein Y and $R^5$ represent, independently, a leaving group and m is as stated above, and the obtained benzodiazepine derivative of the formula IV

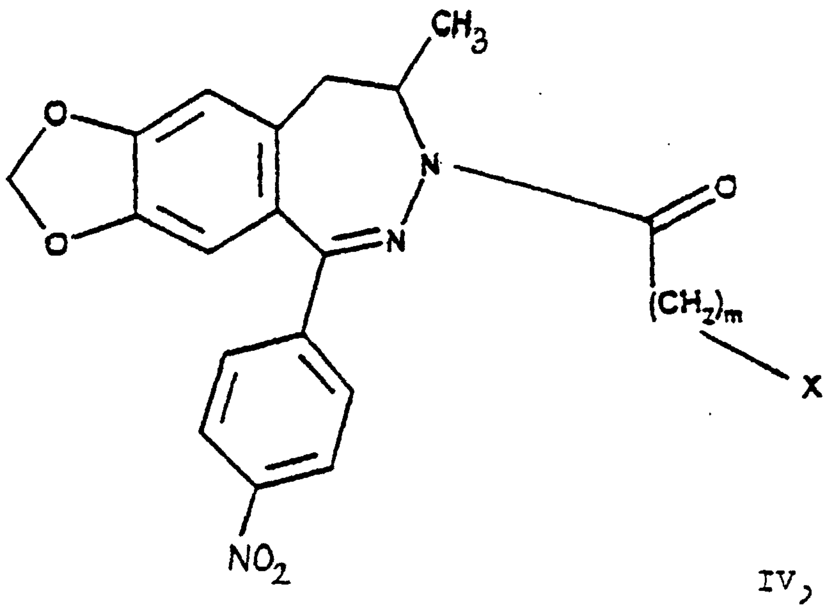

IV,

wherein X stands for a leaving group, m is as stated above, is reacted with an amine of the formula VII

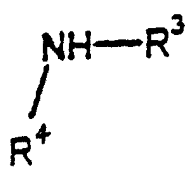

VII ,

wherein $R^3$ and $R^4$ are as stated above and the obtained compound in which instead of the amino group on the phenyl ring of the formula I there is a nitro group and $R^0$, A and B are as defined in connection with the formula I, is transformed into a compound of the formula I by reduction;

or

c) for the preparation of a compound of the formula I, wherein $R^0$ stands for a group of the formula $-(CH_2)_m-R$, in which R represents a halogen atom, m has a value of 0, 1 or 2 and A forms together with B a valence bond, the 8-methyl-5-(4-nitrophenyl)- 9H-1,3-dioxolo/4,5-h//2,3/benzodiazepine of the formula II

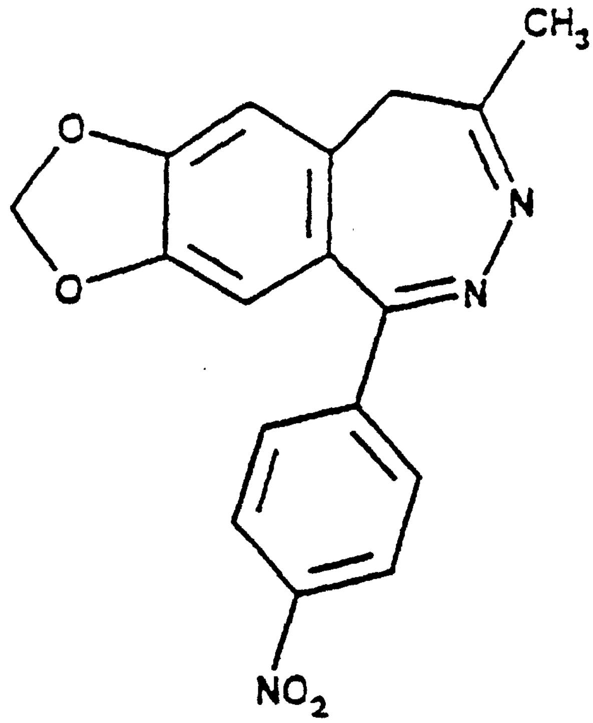

II

is reacted with an acylating agent of the formula IX

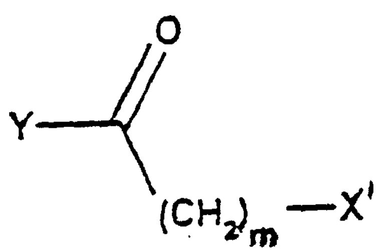

IX,

wherein Y represents a leaving group, X' stands for a halogen atom and m has a value of 0, 1 or 2 and the obtained compound in which instead of the amino group on the phenyl ring of the formula I there is a nitro group and $R^0$, A and B are as defined in connection with the formula I, is transformed into a compound of the formula I by reduction;

or

d) for the preparation of a compound of the formula I, wherein $R^0$ stands for a group of the formula $-(CH_2)_m-R$, in which R represents a group of the formula $-NR^3R^4$, wherein $R^3$, $R^4$ and m are as defined in connection with the formula I and A forms together with B a valence bond, the 8-methyl-5-(4-nitrophenyl)-9H-1,3-dioxolo-/4,5- h//2,3/benzodiazepine of the formula II is reacted with an acylating agent of the formula IX, wherein each of Y and X' represent, independently from each other, a leaving group and m is as stated above, and the obtained acylated compound of the formula VIII

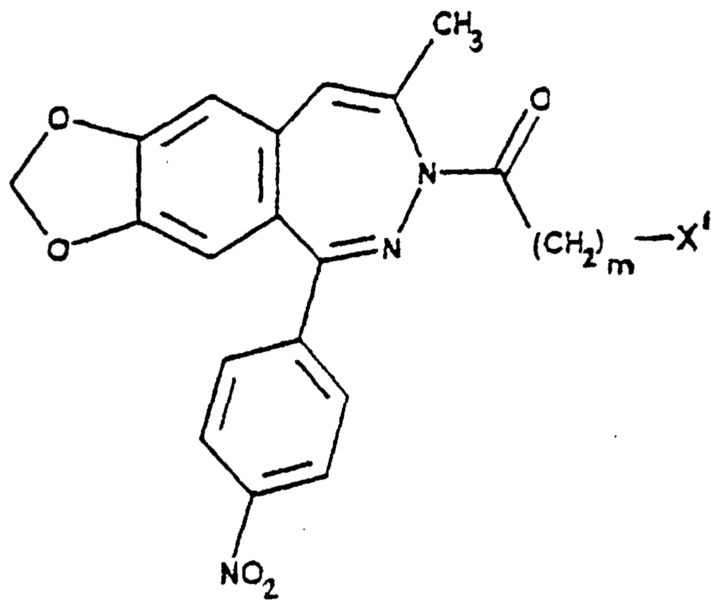

VIII,

wherein X' and m are as defined above, is reacted with an amine of the formula $HNR^3R^4$, wherein $R^3$ and $R^4$ are as stated above; and the obtained compound in which instead of the amino group on the phenyl ring of the formula I there is a nitro group and $R^0$, A and B are as defined in connection with the formula I, is transformed into a compound of the formula I by reduction;
and, if desired, an obtained base of the formula I is converted to a pharmaceutically suitable acid addition salt or liberated from the acid addition salt.

**7.** A pharmaceutical composition comprising a 1,3- dioxolo/4,5-h//2,3/benzodiazepine derivative according to any of claims 1 to 5 as the active ingredient and one or more conventional carrier(s).

**8.** The use of the 1,3-dioxolo/4,5-h//2,3/benzodiazepines according to claim 1 for preparing medicaments for the treatment of epilepsy or a neurodegenerative disease or a state after stroke.

**Patentansprüche**

**1.** 1,3-Dioxolo/4,5-h//2,3-benzodiazepin-Derivat der Formel I

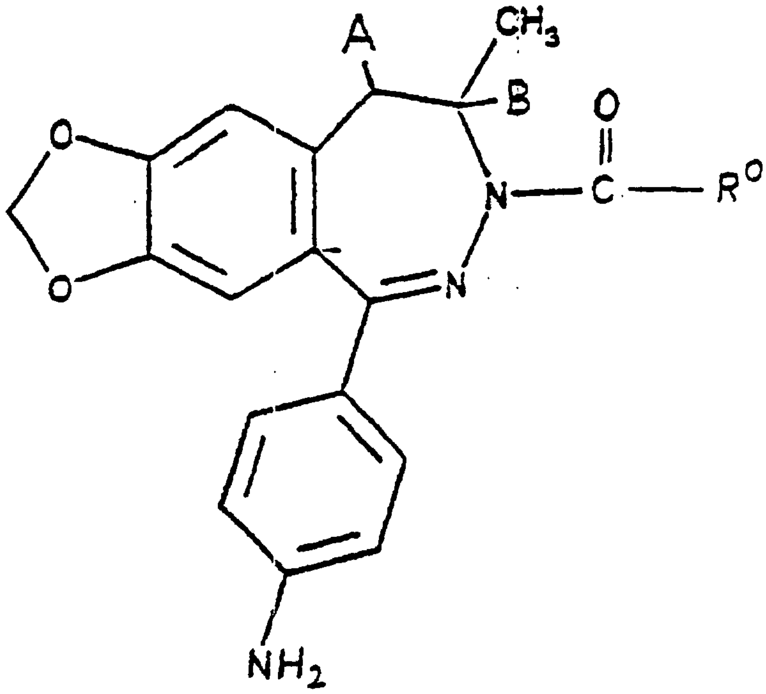

I

worin

a) A und B Wasserstoff bedeuten oder eine Wertigkeitsbindung bilden und

$R^0$ für eine Gruppe der Formel $-(CH_2)_m$-R steht,
worin
R für ein Halogenatom oder eine Gruppe der Formel $-NR^3R^4$ steht,
worin
$R^3$ für Wasserstoff steht und
$R^4$ $C_{3-6}$-Cycloalkyl, $C_{1-4}$-Alkoxy, Amino oder $C_{1-4}$-Alkyl bedeutet, wobei letzteres substituiert ist durch eine gesättigte hydrocyclische Gruppe mit 5 oder 6 Gliedern und 1 Stickstoffatom oder 1 Stickstoffatom und 1 Sauerstoffatom als Heteroatom(e) umfasst,
$R^3$und $R^4$ unabhängig voneinander für $C_{3-6}$-Cycloalkyl, $C_{1-4}$-Alkoxy, Amino oder $C_{1-4}$-Alkyl stehen, wobei letzteres substituiert ist durch eine gesättigte heterocyclische Gruppe mit 5 oder 6 Gliedern und 1 Stickstoffatom oder 1 Stickstoffatom und 1 Sauerstoffatom als Heteroatom(e) umfasst, oder
$R^3$ und $R^4$ zusammen mit dem benachbarten Stickstoffatom und gegebenenfalls mit einem Sauerstoffatom eine gesättigte heterocyclische Gruppe mit 5 oder 6 Gliedern bilden, und m 0, 1 oder 2 ist
oder

b) A und B zusammen eine Wertigkeitsbindung bilden,

$R^0$ für eine Gruppe der Formel $-(CH_2)_m$-R steht, worin
R zusätzlich zu den unter a) definierten Bedeutungen alternativ für eine Gruppe der Formel $-NR^3R^4$ steht, worin
$R^3$ und $R^4$ unabhängig voneinander für Wasserstoff oder nicht substituiertes $C_{1-4}$-Alkyl stehen
und
m 0 ist
oder

c) A und B für Wasserstoff stehen,

$R^0$ für eine Gruppe der Formel $-(CH_2)_m$-R steht, worin

R zusätzlich zu den unter a) definierten Bedeutungen alternativ für eine Gruppe der Formel $-NR^3R^4$ steht, worin
$R^3$ ein Wasserstoffatom bedeutet und
$R^4$ für eine nicht substituierte $C_{2-4}$-Alkylgruppe steht
und
m 0 ist,

und pharmazeutisch geeignete Säureadditionssalze davon.

**2.** 1,3-Dioxolo/4,5-h//2,3/benzodiazepin-Derivat gemäß Anspruch 1, worin
A und B für Wasserstoffatome stehen
und
das Halogenatom, welches durch R repräsentiert sein kann, ein Chloratom ist,
oder
die Cycloalkylgruppe, für welche
$R^3$ und/oder $R^4$ stehen können, [eine] Cyclopropylgruppe(n) ist/sind,
und
m wie in Anspruch 1 definiert ist,
und pharmazeutisch geeignete Säureadditionssalze davon.

**3.** 1,3-Dioxolo/4,5-h//2,3/benzodiazepin-Derivat gemäß Anspruch 1, worin
A und B für Wasserstoffatome stehen,
die Alkylgruppe(n), für welche $R^3$ und/oder $R^4$ stehen können, [eine] $C_{1-2}$-Alkylgruppe(n) ist/sind
und
die heterocyclische Gruppe, durch welche die Alkylgruppe(n), für welche $R^3$ und/oder $R^4$ stehen kann, substituiert sein kann, oder die heterocyclische Gruppe, welche durch $R^3$ und/oder $R^4$ mit dem benachbarten Stickstoffatom und mit einem Sauerstoffatom geformt werden kann, eine Morpholinogruppe ist
und
m einen Wert von 0 oder 1 hat,
und pharmazeutisch geeignete Säureadditionssalze davon.

**4.** 1,3-Dioxolo/4,5-h//2,3/benzodiazepin-Derivat gemäß Anspruch 3, worin
A und B für Wasserstoffatome stehen,
$R^3$ für ein Wasserstoffatom steht,
$R^4$ für eine Cyclopropylgruppe oder eine Aminogruppe steht
und
m einen Wert von 0 besitzt,
und pharmazeutisch geeignete Säureadditionssalze davon.

**5.** 8-Methyl-7H-1,3-dioxolo/4,5-h//2,3/benzodiazepin-Derivat gemäß Anspruch 1, worin
A und B zusammen eine Wertigkeitsbindung bilden
und
das Halogenatom, welches durch R repräsentiert werden kann, ein Chloratom ist,
oder
die Alkylgruppe(n), für welche die heterocyclische Gruppe(n), durch welche die Alkylgruppe(n), für welche $R^3$ und/oder $R^4$ stehen kann, substituiert sein kann, [eine] Morpholinogruppe(n) ist/sind
und
m einen Wert von 0, 1 oder 2 besitzt,
und pharmazeutisch geeignete Säureadditionssalze davon.

**6.** Verfahren zur Herstellung eines 1,3-Dioxolo/4,5-h//2,3/benzodiazepin-Derivats der Formel I, worin A, B und $R^0$ wie in Anspruch 1 definiert sind, gemäß Anspruch 1, und pharmazeutisch geeignete Säureadditionssalze davon, **dadurch gekennzeichnet, dass**

a) für die Herstellung einer Verbindung der Formel I, in der $R^0$ für eine Gruppe der Formel $-(CH_2)_m-R$ steht, worin R für ein Halogenatom steht, m einen Wert von 0, 1 oder 2 hat und A und B für Wasserstoffatome stehen, das 7,8-Dihydro-8-methyl-5-(4-nitrophenyl)-9H-1,3-dioxolo/4,5-h//2,3/benzodiazepin der Formel III

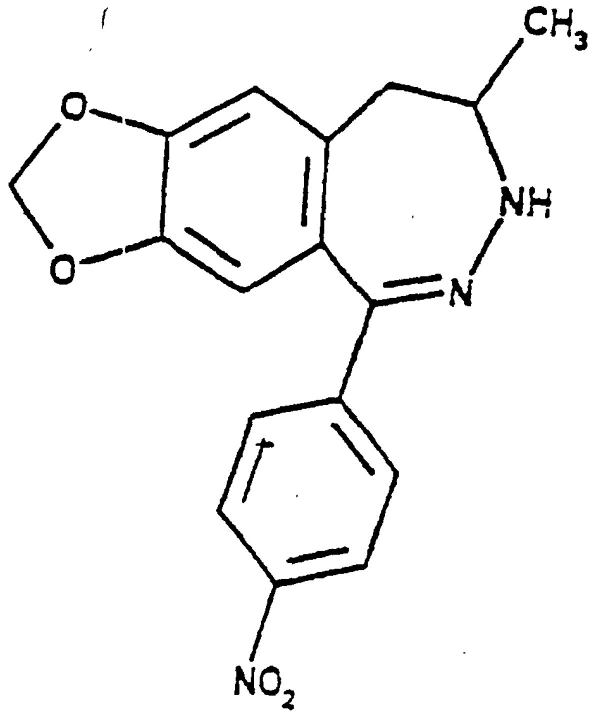

III

mit einem Reagenz der Formel VI

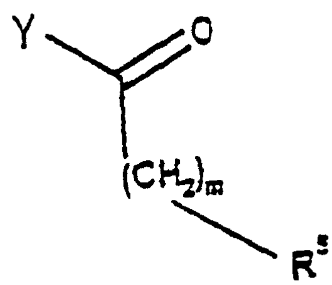

VI

worin Y für eine Abgangsgruppe steht und $R^5$ ein Halogenatom ist, umgesetzt wird und die erhaltene Verbindung, bei der anstelle der Aminogruppe auf dem Phenylring der Formel I eine Nitrogruppe vorliegt und $R^0$, A und B wie in Verbindung der Formel I definiert sind, zu einer Verbindung der Formel I durch Reduktion umgewandelt wird; oder

b) zur Herstellung einer Verbindung I, worin $R^0$ für eine Gruppe der Formel $-(CH_2)_m-R$ steht, worin R für eine Gruppe der Formel $-NR^3R^4$ steht, in der $R^3$, $R^4$ und m wie in Verbindung mit der Formel I definiert sind und A und B für Wasserstoffatome stehen, das 7,8-Dihydro-8-methyl-5-(4-nitrophenyl)-9H-1,3-dioxolo/4,5-h//2,3/benzodiazepin der Formel III mit einem Reagenz der Formel VI umgesetzt wird, wobei Y und $R^5$ unabhängig für eine Abgangsgruppe stehen und m wie oben angegeben ist, und das erhaltene Benzodiazepin-Derivat der Formel IV

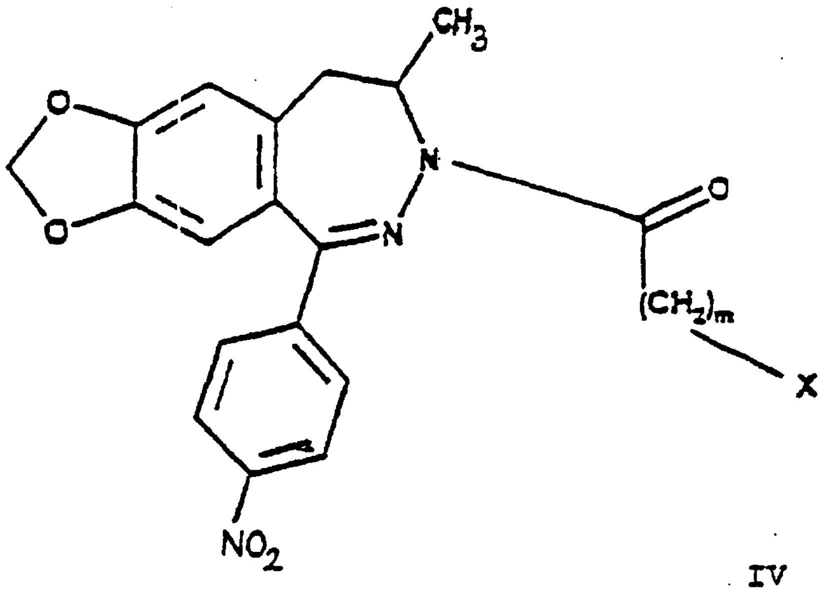

IV

worin X für eine Abgangsgruppe steht, m wie oben angegeben ist, mit einem Amin der Formel VII

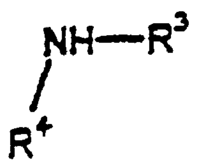

VII

worin $R^3$ und $R^4$ wie oben angegeben sind, umgesetzt wird und die erhaltene Verbindung, in welcher anstelle der Aminogruppe auf dem Phenylring der Formel I eine Nitrogruppe vorliegt und $R^0$, A und B wie in Verbindung mit der Formel I definiert sind, zu einer Verbindung der Formel I durch Reduktion umgewandelt wird; oder

c) für die Herstellung einer Verbindung der Formel I, worin $R^0$ für eine Gruppe der Formel $-(CH_2)_m-R$ steht, in der R für ein Halogenatom steht, m einen Wert von 0, 1 oder 2 besitzt und A zusammen mit B eine Wertigkeitsbindung bildet, das 8-Methyl-5-(4-nitrophenyl)-9H-1,3-dioxolo/4,5-h//2,3/benzodiazepin der Formel II

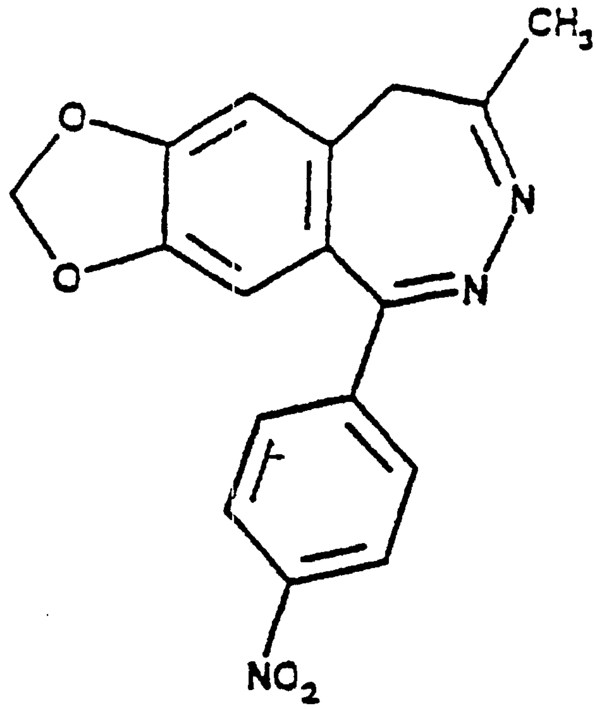

II

mit einem Acylierungsmittel der Formel IX

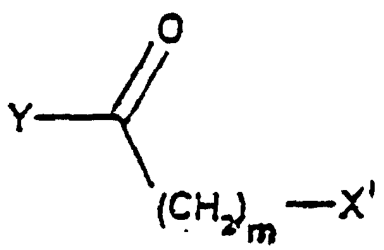

IX

worin Y für eine Abgangsgruppe steht, X' für ein Halogenatom steht und m einen Wert von 0, 1 oder 2 hat, umgesetzt wird, und die erhaltene Verbindung, in der anstelle der Aminogruppe auf dem Phenylring der Formel I eine Nitrogruppe vorliegt und $R_0$, A und B wie in Verbindung mit der Formel I definiert sind, zu einer Verbindung der Formel I durch Reduktion umgewandelt wird; oder

d) zur Herstellung einer Verbindung der Formel I, worin $R^0$ für eine Gruppe der Formel $-(CH_2)_m-R$ steht, in der R für eine Gruppe der Formel $-NR^3R^4$ steht, in der $R^3$, $R^4$ und m wie in Verbindung mit der Formel I definiert sind und A zusammen mit B eine Wertigkeitsbindung bildet, das 8-Methyl-5-(4-nitrophenyl)-9H-1,3-dioxolo/4,5-h//2,3/benzodiazepin der Formel II mit einem Acylierungsmittel der Formel IX umgesetzt wird, wobei jedes von Y und X' unabhängig voneinander eine Abgangsgruppe bedeuten und m wie oben angegeben definiert ist, und die erhaltene acylierte Verbindung der Formel VIII

1

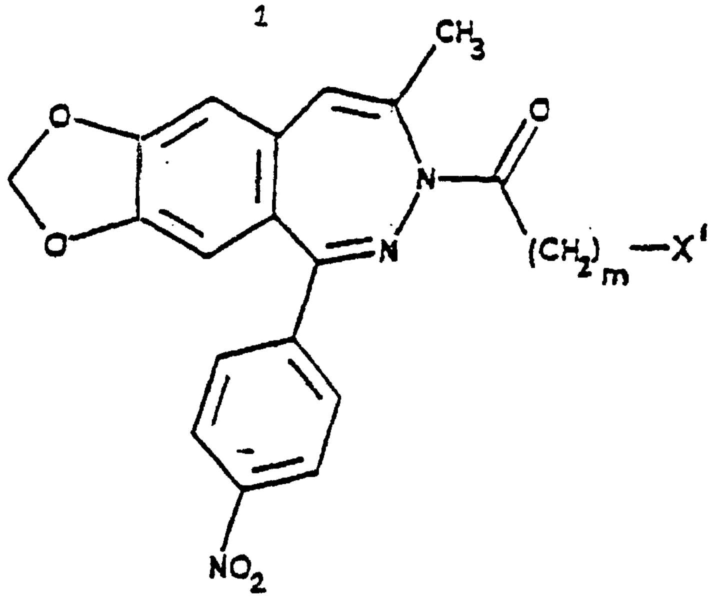

VIII

in der X' und m wie oben definiert sind, mit einem Amin der Formel $HNR^3R^4$, worin $R^3$ und $R^4$ wie oben definiert sind, umgesetzt wird; und die erhaltene Verbindung, in der anstelle der Aminogruppe auf dem Phenylring der Formel I eine Nitrogruppe vorliegt und $R^0$, A und B wie in Verbindung mit der Formel I definiert sind, zu einer Verbindung der Formel I durch Reduktion umgewandelt wird;
und, sofern erwünscht, eine erhaltene Base der Formel I zu einem pharmazeutisch geeigneten Säureadditionssalz umgewandelt wird oder aus dem Säureadditionssalz freigesetzt wird.

**7.** Pharmazeutische Zusammensetzung, umfassend ein 1,3-Dioxolo/4,5-h//2,3/-benzodiazepin-Derivat gemäß mindestens einem der Ansprüche 1 bis 5, als aktiven Bestandteil und einen oder mehrere herkömmliche Träger.

**8.** Verwendung des 1,3-Dioxolo/4,5-h//2,3/-benzodiazepins gemäß Anspruch 1 zur Herstellung von Medikamenten zur Behandlung von Epilepsie oder einer neurodegenerativen Erkrankung oder einem Zustand nach Schlaganfall.

## Revendications

**1.** Dérivé de 1,3-dioxolo/4,5-h//2,3/benzodiazépine de formule I

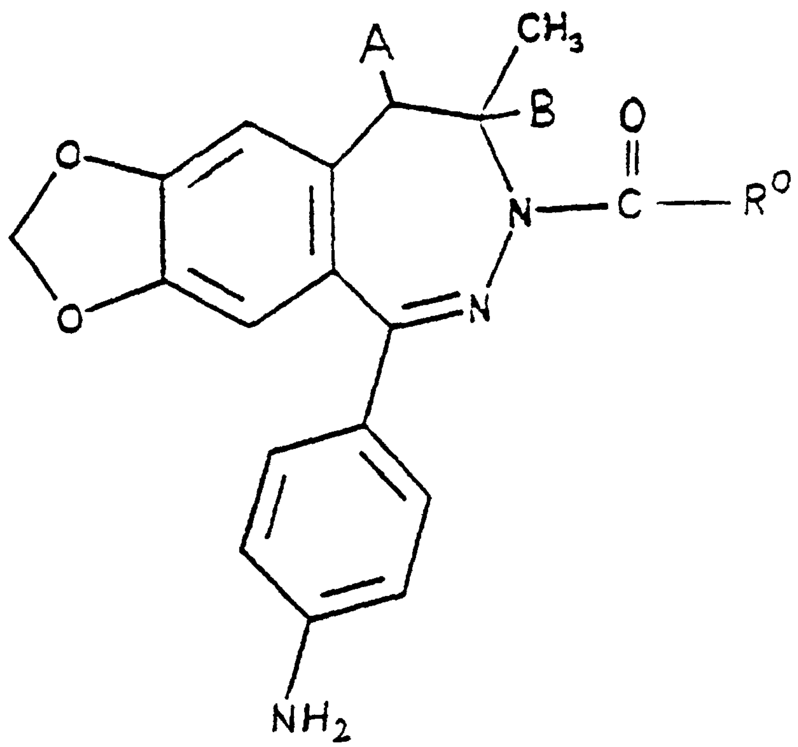

I ,

dans laquelle

a) A et B représentent un hydrogène ou forment ensemble une liaison de valence et

$R^0$ correspond à un groupe de formule $-(CH_2)_m-R$,
dans laquelle
R représente un atome d'halogène ou un groupe de formule $-NR^3R^4$,
dans laquelle
$R^3$ représente un hydrogène et
$R^4$ signifie un cycloalkyle en $C_3$ à $C_6$, un alcoxy en $C_1$ à $C_4$, un amino ou un alkyle en $C_1$ à $C_4$, qui est plus tard substitué par un groupe hétérocyclique saturé ayant 5 ou 6 membres et comprenant 1 atome d'azote ou 1 atome d'azote et 1 atome d'oxygène comme hétéroatome(s),
$R^3$ et $R^4$ signifient, indépendamment l'un de l'autre, un cycloalkyle en $C_3$ à $C_6$, un alcoxy en $C_1$ à $C_4$, un amino ou un alkyle en $C_1$ à $C_4$, qui est substitué plus tard par un groupe hétérocyclique saturé ayant 5 ou 6 membres et comprenant 1 atome d'azote ou 1 atome d'azote et 1 atome d'oxygène comme hétéroatome(s),
ou
$R^3$ et $R^4$ forment ensemble avec l'atome d'azote adjacent et éventuellement avec un atome d'oxygène, un groupe hétérocyclique saturé ayant 5 ou 6 membres
et
m vaut 0, 1 ou 2
ou
b) A et B forment ensemble une liaison de valence, $R^0$ correspond à un groupe de formule $-(CH_2)_m-R$, dans laquelle
R, en plus des significations définies en a) représente en variante un groupe de formule $-NR^3R^4$,
dans laquelle
$R^3$ et $R^4$ signifient, indépendamment l'un de l'autre, un hydrogène ou un alkyle en $C_1$ à $C_4$ non substitué

et
m vaut 0
ou
c) A et B représentent un hydrogène,
$R^0$ correspond à un groupe de formule $-(CH_2)_m-R$,
dans laquelle
R, en plus des significations définies en a), représente en variante un groupe de formule $-NR^3R^4$, dans laquelle
$R^3$ signifie un atome d'hydrogène et $R^4$ correspond à un groupe alkyle en $C_2$ à $C_4$ non substitué
et
m vaut 0,

et leurs sels d'addition acides pharmaceutiquement acceptables.

**2.** Un dérivé de 1,3-dioxolo/4,5-h//2,3/benzodiazépine selon la revendication 1, dans lequel
A et B représentent des atomes d'hydrogène
et
l'atome d'halogène qui peut être représenté par R est un atome chloro
ou
le groupe cycloalkyle pour lequel
$R^3$ et/ou $R^4$ peuvent correspondre, est/sont un(des) groupe(s) isopropyle et
m est tel que défini dans la revendication 1,
et leurs sels d'addition acides pharmaceutiquement acceptables.

**3.** Dérivé de 1,3-dioxolo/4,5-h//2,3/benzodiazépine selon la revendication 1, dans lequel
A et B représentent des atomes d'hydrogène,
le(s) groupe(s) alkyle pour le(s)quel(s) $R^3$ et/ou $R^4$ peuvent correspondre, est/sont un(des) groupe(s) alkyle en $C_1$ à $C_2$
et
le groupe hétérocyclique par lequel le(s) groupe(s) alkyle, pour le(s)quel(s) $R^3$ et/ou $R^4$ peuvent correspondre, peut être substitué ou le groupe hétérocyclique qui peut être formé par $R^3$ et/ou $R^4$ avec l'atome d'azote adjacent et avec un atome d'oxygène est un groupe morpholino
et
m a une valeur de 0 ou 1,
et leurs sels d'addition acides pharmaceutiquement acceptables.

**4.** Dérivé de 1,3-dioxolo/4,5-h//2,3/benzodiazépine selon la revendication 3, dans lequel
A et B représentent des atomes d'hydrogène,
$R^3$ représente un atome d'hydrogène,
$R^4$ correspond à un groupe cyclopropyle ou à un groupe amino
et
m a une valeur de 0
et leurs sels d'addition acides pharmaceutiquement acceptables.

**5.** Dérivé de 8-méthyl-7H-1,3-dioxolo/4,5-h//2,3/benzodiazépine selon la revendication 1, dans lequel
A et B forment ensemble une liaison de valence,
et
l'atome d'halogène qui peut être représenté par R est un atome chloro
ou
le(s) groupe(s) alkyle pour le(s)quel(s) le(s) groupe(s) hétérocyclique(s), par le(s)quel(s) le(s) groupe(s) alkyle, pour le(s)quel(s) $R^3$ et/ou $R^4$ peuvent correspondre, peut être substitué, est/sont un (des) groupe(s) morpholino
et m a une valeur de 0, 1 ou 2,
et leurs sels d'addition acides pharmaceutiquement acceptables.

**6.** Procédé pour la préparation d'un dérivé de 1,3-dioxolo/4,5-h//2,3/benzodiazépine de formule I, dans laquelle A, B et $R^0$ sont tels que définis dans la revendication 1, selon la revendication 1, et leurs sels d'addition acides pharmaceutiquement acceptables, **caractérisé en ce que**

a) pour la préparation d'un composé de formule I, dans laquelle $R^0$ représente un groupe de formule $-(CH_2)_m$-R, dans laquelle R correspond à un atome d'halogène, m a une valeur de 0, 1 ou 2 et A et B représentent des atomes d'hydrogène, la 7,8-dihydro-8-méthyl-5-(4-nitrophényl)-9H-1,3-dioxolo/4,5-h//2,3/benzodiazépine de formule III

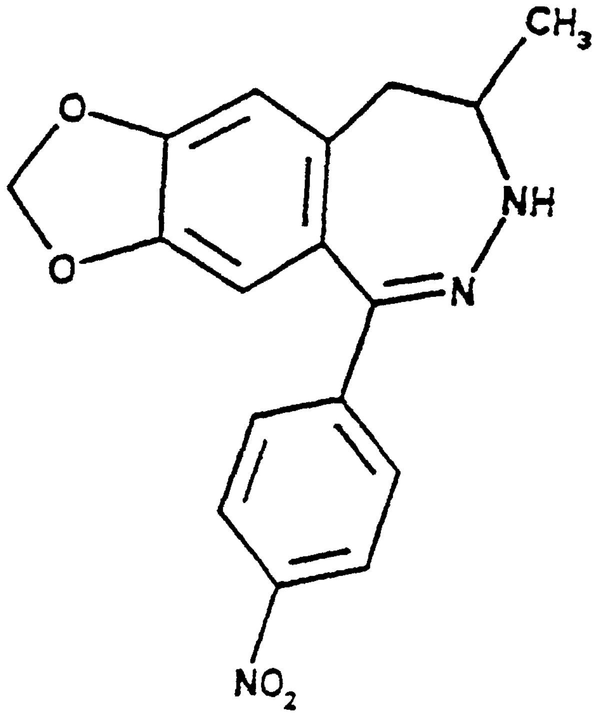

III

est mise à réagir avec un réactif de formule VI

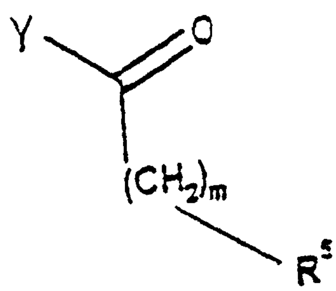

VI ,

dans laquelle Y représente un groupe partant et $R^5$ est un atome d'halogène, et le composé obtenu dans lequel à la place du groupe amino sur le cycle phényle de la formule I, il y a un groupe nitro et $R^0$, A et B sont tels que définis en rapport avec la formule I, est transformé en un composé de formule I par réduction ;

ou

b) pour la préparation d'un composé de formule I, dans laquelle $R^0$ représente un groupe, de formule $-(CH_2)_m$-R, dans laquelle R correspond à un groupe de formule $-NR^3R^4$, dans laquelle $R^3$, $R^4$ et m sont tels que définis en rapport avec la formule I, et A et B représentent des atomes d'hydrogène, la 7,8-dihydro-8-méthyl-5-(4-nitrophenyl)-9H-1,3-dioxolo/4,5-h//2,3/ benzodiazépine de formule III est mise à réagir avec un réactif de formule VI, dans laquelle Y et $R^5$ représentent, indépendamment, un groupe partant et m est comme établi ci-dessus, et le dérivé de benzodiazépine obtenu, de formule IV,

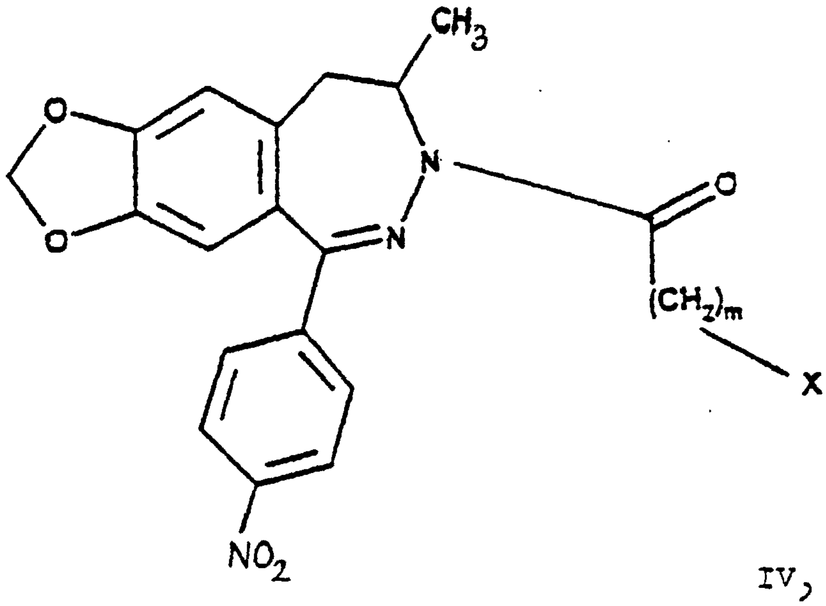

IV,

dans laquelle X correspond à un groupe partant, m est comme présenté ci-dessus, est mise à réagir avec une amine de formule VII

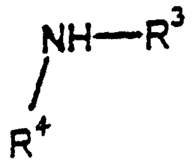

VII ,

dans laquelle $R^3$ et $R^4$ sont comme présentés ci-dessus et le composé obtenu dans lequel à la place du groupe amino sur le cycle phényle de formule I, il y a un groupe nitro et $R^0$, A et B sont tels que définis en rapport avec la formule I, est transformé en un composé de formule I par réduction ;

c) pour la préparation d'un composé de formule I, dans laquelle $R^0$ correspond à un groupe de formule $-(CH_2)_m-R$, dans laquelle R représente un atome d'halogène, m a une valeur de 0, 1 ou 2 et A forme ensemble avec B une liaison de valence, la 8-méthyl-5-(4-nitrophényl)-9H-1,3-dioxolo/4,5-h//2,3/benzodiazépine de formule II

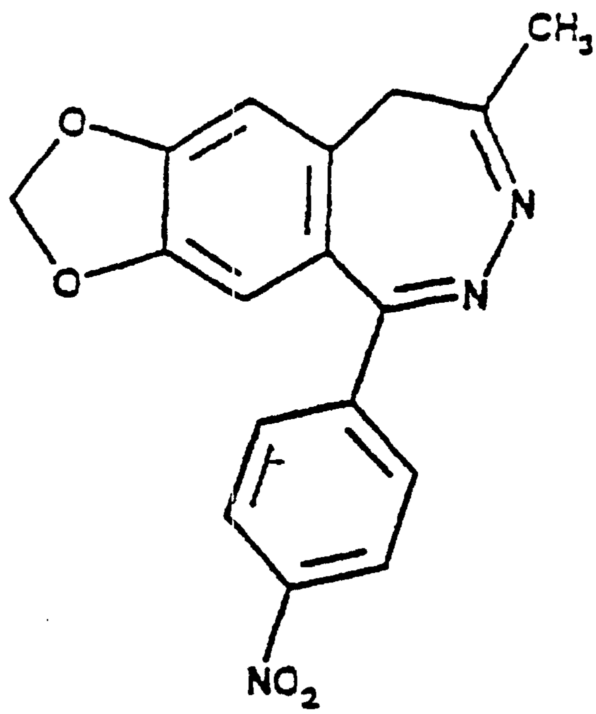

II

est mise à réagir avec un agent acylant de formule IX

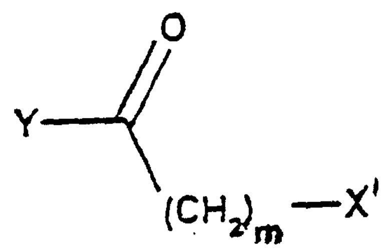

IX,

dans laquelle Y représente un groupe partant, X' correspond à un atome d'halogène et m a une valeur de 0, 1 ou 2, et le composé obtenu dans lequel à la place du groupe amino sur le cycle phényle de formule I, il y a un groupe nitro et $R^0$, A et B sont tels que définis en rapport avec la formule I, est transformé en un composé de formule I par réduction ;

ou

d) pour la préparation d'un composé de formule I,

dans laquelle $R^0$ correspond à un groupe de formule $-(CH_2)_m-R$, dans laquelle R représente un groupe de formule $-NR^3R^4$, dans laquelle $R^3$, $R^4$ et m sont tels que définis en rapport avec la formule I et A forme ensemble avec B une liaison de valence, la 8-méthyl-5-(4-nitrophényl)-9H-1,3-dioxolo/4,5-h//2,3/benzodiazépine de formule II est mise à réagir avec un agent acylant de formule IX, dans laquelle chacun de Y et X' représentent, indépendamment l'un de l'autre, un groupe partant et m est tel que défini ci-dessus, et le composé acylé obtenu de formule VIII

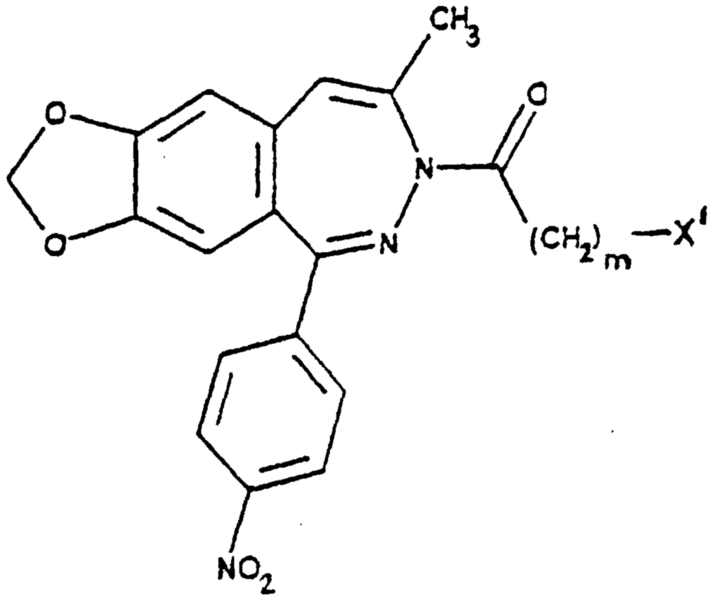

VIII ,

dans laquelle X' et m sont tels que définis ci-dessus, est mis à réagir avec une amine de formule $HNR^3R^4$, dans laquelle $R^3$ et $R^4$ sont tels que définis ci-dessus ; et le composé obtenu, dans lequel à la place du groupe amino sur le cycle phényle de formule I, il y a un groupe nitro, et $R^0$, A et B sont tels que définis en rapport avec la formule I, est transformé en un composé de formule I par réduction ;

et, si on le souhaite, une base obtenue de formule I est convertie en un sel d'addition acide pharmaceutiquement acceptable ou libérée du sel d'addition acide.

**7.** Composition pharmaceutique comprenant un dérivé de 1,3-dioxolo/4,5-h//2,3/benzodiazépine selon l'une des revendications 1 à 5 comme ingrédient actif et un ou plusieurs porteurs conventionnels.

**8.** Utilisation des 1,3-dioxolo/4,5-h//2,3/benzodiazépines selon la revendication 1, pour préparer des médicaments pour le traitement de l'épilepsie ou d'une maladie neurodégénérative ou d'un état postérieur à un accident vasculaire cérébral.